# EUROPEAN PATENT SPECIFICATION

(11) **EP 1 712 248 B1**
(45) Date of publication and mention of the grant of the patent: **27.11.2013**
(21) Application number: 06014650.3
(22) Date of filing: 08.06.2000
(51) Int. Cl.: A61M 25/00, A61B 17/34

(54) **Multiple lumen access device**
Mehrlumige Zugangsvorrichtung
Dispositif d'accès à plusieurs lumières

(30) Priority: 08.06.1999 US 329002
(43) Date of publication of application: 18.10.2006
(62) Divisional of application: 00942709.7
(73) Proprietor: Edwards Lifesciences Corporation, Irvine, CA 92614 (US)
(72) Inventor: Mooney, Charles R., Costa Mesa California 92626 (US); Pecor, Robert, Aliso Viejo California 92656 (US); Bobo, Donald E. Jr., Santa Ana California 92706 (US); Higgins, MIchael J., Trabuco Canyon California 92679 (US); Miraki, Manouchehr A., Laguna Hills California 92653 (US); Bulman, Erik E., Mission Viejo California 92694 (US); Willoughby, Gary R., Castle Rock Colorado 80104 (US)
(74) Representative: Potter Clarkson LLP

(56) References cited:
- EP-A- 0 616 817
- EP-A- 0 738 520
- WO-A-98/24501
- WO-A-99/20326
- US-A- 4 670 009
- US-A- 5 156 596
- US-A- 5 207 648
- US-A- 5 601 603
- US-A- 5 718 692
- US-A- 5 728 064
- US-A- 5 800 414

## Description

### RELATED APPLICATIONS

The present application is a continuation-in-part of co-pending U.S. Application Serial No. 08/953,105, filed October 17, 1997, which is a continuation-in-part of U.S. Application Serial No. 08/756,763, filed November 26, 1996 under the same title, abandoned.

### BACKGROUND OF THE INVENTION

### Field of the Invention

The present invention relates generally to medical devices which are used to provide access into the human body. More particularly, the present invention is directed to access devices which provide a single, relatively long-term, entry port into the body. The entry port is used by doctors and other medical professionals to selectively introduce a variety of medical implements and fluids into the body and for *in vivo* diagnostic testing and other treatment protocols.

### Description of Related Art

A wide variety of medical devices have been developed in recent years for providing access into the human blood stream. WO 98/24501 A describes such a device. These devices have traditionally been divided into two different groups based on their function and purpose. The first group of devices includes catheters which are designed to introduce therapeutic and/or diagnostic fluids into the blood stream. The second group includes devices commonly referred to as "introducers" which are designed to provide an intermediate term access port into the body through which various medical implements may be passed for therapeutic and/or diagnostic purposes. As a generalization, catheters are longer and more flexible than introducers.

Central venous catheters are relatively long tubular devices which have tapered distal tips which are designed for entry into central veins to provide a dedicated route of fluid infusion into the body. The original venous catheters were single lumen devices which provided the ability to infuse a single liquid into the vein at one time. Multiple lumen catheters have since been developed which allow simultaneous introduction of two or more liquids into the vein. The central venous pressure catheter is a type of common multiple lumen catheter which allows the simultaneous introduction and withdrawal of fluids as well as the capability of monitoring blood pressure and other vital parameters. The portion of the catheter which remains outside of the body has been continually refined and redesigned to provide a low profile which increases comfort and reduces the awkwardness associated with a dedicated tube exiting the body.

Introducers are substantially different from catheters in both design and purpose. An introducer is an access device which is intended to provide a dedicated access port into the body. Catheters, on the other hand, are intended to be used to infuse or withdraw fluids from the body. Introducers typically include a relatively short lumen through which various medical implements, including catheters, can be selectively introduced and removed from the body. An important feature of any introducer is the valve assembly. The valve assembly provides a constant seal between the blood stream and the *in vitro* environment as medical implements are introduced and withdrawn from the body. The valve assembly is typically located outside of the body at the proximal end of the introducer. As a result, the proximal end of introducers has tended to be relatively bulky.

In addition to a valve assembly, many introducers include a side arm at the proximal end. The side arm is connected to the lumen so that fluids can be introduced into the body simultaneously with the medical device. The introducer lumen is considered to be a "shared" lumen in that the lumen provides a common conduit for both medical implements and fluid pharmaceuticals or diagnostics.

The currently available introducers and other access devices are well-suited for their intended purpose. However, new medical treatments and diagnostic procedures are continually being developed which require more versatile access into the body. For example, organ transplant procedures and cardiac angioplasty require the introduction of complex combinations of medical implements and diagnostic/therapeutic fluids into the body. Many of the presently available access devices are not well-suited for these relatively complex procedures. As a result, multiple access devices are required which must be located at multiple access sites necessitating multiple entry punctures. Accordingly, there is a continuing need to provide improved access devices that have additional capabilities which increase their versatility and usefulness for the increasing variety of invasive treatments and procedures.

### SUMMARY OF THE INVENTION

In accordance with the present invention as claimed, an improved access device is provided which is designed to provide selective introduction of medical implements into the body while simultaneously providing auxiliary access through dedicated multiple lumens. The present invention is an improvement over existing introducers and other access devices in that multiple lumen access is provided through the introducer in addition to the shared lumen which is used for both medical implements and fluid pharmaceuticals or diagnostics. As an advantage, the improved access device reduces the number of devices required to introduce multiple implements and fluids into the body during complex surgical and diagnostic procedures.

The present invention desirably includes a multiple lumen access system for use in providing an entry port into the human body for selectively introducing medical implements therethrough and for providing simultaneous auxiliary access into the body. The system includes a multiple lumen access device comprising an outer tube which has a distal end for introduction into the body and a cross-sectional area. A device lumen through which medical implements may be passed is defined within the cross-sectional area of the outer tube, the device lumen having a distal end and a proximal end. At least one auxiliary lumen is defined within the cross-sectional area and separately from the device lumen, the auxiliary lumen having a distal end and proximal end. Finally, a device lumen valve is associated with the proximal end of the device lumen to provide sealing of the device lumen when medical implements are both present and absent from the device lumen. Such device lumen valve may be separate and detachable or it may be integral with the system.

A multiple lumen access system according to the present invention may also include a junction housing having a proximal end and a distal end to which the proximal end of the outer tube connects. The junction housing includes a main channel in fluid communication with the device lumen and at least one auxiliary channel in fluid communication with the at least one auxiliary lumen, the main channel and auxiliary channel(s) diverging from the outer tube to be non-intersecting in the junction housing.

In one embodiment, the device lumen valve is provided as a part of the junction housing and is in fluid communication with the main channel. A device channel may be formed in the junction housing at an angle with the main channel and terminating at an internal end in fluid communication with the main channel. The device lumen valve may be positioned at an external end of the device channel so that medical devices may be inserted therethrough and enter the main channel at an angle. The main channel desirably may continue from the distal end of the junction housing past the device lumen to an opening in the junction housing enabling introduction of fluids therethrough to the main channel. In one embodiment, the device lumen valve is molded separately from the junction housing of a material more rigid than the junction housing and is assembled with the multiple lumen access device by insertion in a cavity formed in the junction housing.

In an alternative embodiment, the main channel and auxiliary channel(s) of the junction housing may be oriented substantially coplanar so that the junction housing is substantially flat, the system further including an extension tube extending from the proximal end of the junction housing and in fluid communication with the main channel wherein the device lumen valve is connected to the extension tube to therefore be in fluid communication with the main channel. A side port in the device lumen valve may be provided enabling infusion of fluids to the extension tube and main channel. Furthermore, mating threaded connectors may be included between the device lumen valve and the extension tube enabling easy removal of the device lumen valve. Any appropriate connector, for example a luer connector, may be provided on the device lumen valve, and the system may also include an infusion syringe having a mating luer connector.

Further, in one embodiment, a multiple lumen access device may be provided with a multi-lumen sheath, a junction housing coupled to the multi-lumen sheath and a strain relief insert coupled to the junction housing. The strain relief insert is formed of a soft bendable material capable of flexing to prevent multi-lumen sheath from kinking at the sheath/junction housing coupling. In further embodiment, the multiple lumen access device is formed by coupling a single lumen catheter to a junction housing having a main channel and at least one auxiliary channel through a multi-function adapter.

In another embodiment, the present invention is directed to a multiple lumen access device including an outer tube which has a distal end for introduction into the body and a proximal end which remains outside of the body. The outer tube may have an exterior surface and an interior surface, the interior surface defining an access passageway which has a cross-sectional area which may vary at different locations between the distal and proximal ends of the outer tube. One or more inner walls are located within the access passageway. The inner wall may form an inner tube that surrounds a device lumen through which medical implements may be inserted into the body. At least one auxiliary lumen is located between the exterior surface of the inner wall and the interior surface of the outer tube.

As another feature of the present invention, two or more auxiliary passageways defined by the interior surface of the outer tube and the exterior surface of the inner walls. The provision of two or more auxiliary passageways allows introduction of additional diagnostics or pharmaceutical liquids simultaneously with introduction of a medical implement through the device lumen. Embodiments of the present invention are also described wherein a single auxiliary lumen is provided.

As a further desirable feature of the present invention the inner walls are sufficiently flexible to be movable from a relaxed position to expanded or contracted positions. The device lumen has a first cross-sectional area in the relaxed position, and in the expanded or contracted positions has cross-sectional areas which are greater than or less than the first cross-sectional area, and less than the cross-sectional area of the access passageway. The flexibility of the inner walls is advantageous in that it allows the insertion of a variety of medical implements having different cross-sectional areas. This flexibility allows the cross-sectional areas and resultant potential fluid flow rate for the auxiliary lumens and the device lumen to be controlled as desired and maximized within the confines of the access passageway.

As an additional feature of the present invention, spacer ribs are provided, for example, on the interior surface of the outer tube. The spacer ribs are located within the auxiliary lumens to prevent complete closure of the lumens during insertion of relatively large medical implements into the device lumen. The spacer ribs located on the surface of the inner wall insure that there is a passageway around devices located within the device lumen.

An alternative multiple lumen access device of the present invention comprises a tubular single lumen sheath having at least one infusion port and an elongated implement sized to fit coaxially within the single lumen sheath and form multiple independent lumens, and when at least one of the lumens is in fluid communication with the infusion port. The elongated implement may be formed from a sufficiently flexible material so that at least one lumen formed by the sheath and the implement has flexible walls movable from a relaxed to a flexed positions. Another alternative multiple lumen access device comprises a multi-lumen catheter having a main lumen tube, at least one side lumen tube connected in a side-by-side fashion with the main lumen tube and being peelable from the main lumen to form sidearms, and a hub connected to the main lumen tube and side lumen tube for fluid delivery or passage of a medical device therethrough.

A method for introducing medical devices into the body through a single entry port while allowing simultaneous introduction of other devices, implements or fluids through the use of the multiple lumen access device of the present invention may include the steps of providing a multiple lumen access device in accordance with the present invention having at least one flexible wall; introducing the multiple lumen access device into the body with the distal ends of the device lumen and the auxiliary lumen being positioned within a vasculature of the body; and flowing a medical solution through the auxiliary lumen to move the flexible wall from the relaxed position to a flexed position.

The method may include the steps of providing a tubular single lumen sheath having proximal and distal ends, at least one infusion port being provided on the proximal end of the sheath; providing an elongated implement sized to fit coaxially within the single lumen sheath, at least one of the lumens being in fluid communication with the infusion port; inserting the elongated implement into the single lumen sheath to form multiple independent lumens therein; and flowing a medical solution through one or more of the multiple independent lumens.

The above-described and many other features and attendant advantages of a multiple lumen access device will become better understood by reference to the following detailed description when taken in conjunction with the accompanying drawings.

### BRIEF DESCRIPTION OF THE DRAWINGS

FIG. 1 is a perspective view of an exemplary multiple lumen access device.
FIG. 2 is a sectional view of FIG. 1 taken in the 2-2 plane of FIG. 1.
FIG. 3A is a sectional view taken in the same 2-2 plane of FIG. 1 which shows a relatively small diameter medical device located within the device lumen.
FIG. 3B is a sectional view taken in the same 2-2 plane of FIG. 1 showing a relatively large diameter medical implement located within the device lumen.
FIG. 4 is a sectional view of FIG. 1 taken in the 4-4 plane.
FIG. 5 is a sectional view of FIG. 1 taken in the 5-5 plane.
FIG. 6 is a perspective view of an exemplary embodiment.
FIG. 7 is a sectional view of FIG. 6 taken in the 7-7 plane.
FIG. 8 is a sectional view of FIG. 6 taken in the 8-8 plane.
FIG. 9 is a sectional view of a preferred exemplary flexible inner wal showing the location of spacing ribs.
FIG. 10 is a sectional view of a preferred exemplary multiple lumen access device having a single auxiliary lumen.
FIGS. 11A-C are sectional views of an exemplary multiple lumen access device showing a relatively small diameter medical implement located in a central device lumen and the inner walls in relaxed conditions (11A), partially collapsed about the implement due to pressurization of side auxiliary lumens (11B), and substantially completely collapsed about the implement (11C).
FIG. 12 is a graph illustrating an increase in the cross-sectional area (in gauge size) of an auxiliary lumen, such as in the cross-section shown in FIGS. 11A-11C, as the differential pressure between the auxiliary lumen and the device lumen changes.
FIG. 13 is a sectional view of an alternativemulti-lumen sheath having a device lumen on one side and two side-by-side auxiliary lumens.
FIG. 14 is a sectional view of an alternative multi-lumen sheath having a device lumen on one side and two stacked auxiliary lumens.
FIG. 15 is a sectional view of an alternative multi-lumen sheath having no flexible walls therein.
FIG. 16 is a perspective view of a further embodiment of a multiple lumen access device.
FIG. 17 is a perspective sectional view of FIG. 16 taken in the 17-17 plane.
FIG. 18A is a perspective view of an extrusion die for making a sheath portion of a multiple lumen access device.
FIG. 18B is an end view of a sheath portion of the multiple lumen access device as extruded from the die shown in FIG. 18A.
FIGS. 18C and 18D are isolated views of inner extrusion molds of the die shown in FIG. 18A with exemplary dimensions for the sheath portion cross-section called out.
FIG. 19 is an enlarged perspective view of a junction housing of the device shown in FIG. 16.
FIG. 20 is an enlarged perspective of the junction housing of FIG. 19 with a portion cut away on the longitudinal axis.
FIG. 21 is a perspective assembled view of a valve insert used in the junction housing of FIG. 16.
FIG. 22 is an exploded perspective view of the valve insert of FIG. 21.
FIGS. 23A and 23B are two perspective views of a multiple lumen access device similar to that shown in FIG. 16.
FIG. 24 is an elevational view of the multiple lumen access device of FIGS. 23A/23B in place in the vasculature of a patient.
FIG. 25A is a perspective view of a junction housing of the device shown in FIGS. 23 and 24 showing a valve insert and strain relief insert both exploded therefrom.
FIG. 25B is a reversed perspective view of the strain relief insert adapted to be coupled to the junction housing in FIG. 25A.
FIG. 26 is an exploded elevational view of the valve insert shown in FIG. 25A.
FIG. 27 is a perspective view of a clamp portion for the valve insert of FIG. 26.
FIGS. 28A and 28B are perspective views of an adapter which mates with the valve inserts of FIGS. 21 or 26.
FIG. 29 is an elevational view of a multiple lumen access device.
FIG. 30 is a plan view of the multiple lumen access device of FIG. 29 showing more details of an associated catheter system.
FIG. 31 is a perspective view of a proximal end of a low-profile junction housing of the device of FIG. 29.
FIG. 32 is a plan view of an alternative multiple lumen access device with a low profile junction housing.
FIG. 33 is a detailed view of an alternative introducer valve assembly for use in the device of FIGS. 30 or 32.
FIG. 34 is a plan view of an alternative multiple lumen access having a multi-lumen infusion catheter interfacing with a single lumen introducer.
FIGS. 35A-35D are schematic sectional views of sheath/lumen configurations for use in the multi-lumen infusion catheter of FIG. 34.
FIG. 36 is a sectional view of a junction housing used in the device of FIG. 34.
FIG. 37 is an elevational view of a further embodiment of a multi-lumen sheath for use in the device of FIG. 34.
FIG. 38 is a sectional view of the multi-lumen sheath of FIG. 37.
FIG. 39 is a plan view of a multiple lumen access device having a center tube and two side lumen tubes.
FIGS. 40A and 40B are sectional views of a sheath of the multiple lumen access device of FIG. 39 taken along lines 40A-40A and 40B-40B, respectively.
FIG. 41 is an alternative multiple lumen access device with discrete tubes as in FIG. 39 and having a junction housing.
FIG. 42A is an exploded view of a multiple lumen access device having an introducer connected to a multi-lumen catheter by an adjustable adapter.
FIG. 42B is an assembled view of the multiple lumen access device of FIG. 42A.
FIG. 43A is an exploded view of a multiple lumen access device having an introducer with infusion port connected to a multi-lumen catheter by an adapter.
FIG. 43B is an assembled view of the multiple lumen access device of FIG. 43A.
FIG. 44A is an exploded view of a multiple lumen access device having an introducer with infusion port connected to a triple lumen junction housing and obturator by an adapter.
FIG. 44B is an assembled view of the multiple lumen access device of FIG. 44A.
FIG. 45A is an exploded view of a multiple lumen access device having an introducer connected to triple lumen junction housing by a threaded adapter.
FIG. 45B is an assembled view of the multiple lumen access device of FIG. 45A.
FIG. 46A is an exploded view of a multiple lumen access device having an introducer connected to triple lumen junction housing and elongated infusion tube by a threaded adapter.
FIG. 46B is an assembled view of the multiple lumen access device of FIG. 46A.
FIG. 47A is an exploded view of a multiple lumen access device in accordance with the present invention having an introducer with infusion port telescopically fitting within a larger introducer.
FIG. 47B is an assembled view of the multiple lumen access device of FIG. 47A.
FIG. 48A is an exploded view of a multiple lumen access device with a multi-ribbed hollow obturator telescopically fitting within an introducer with infusion ports.
FIG. 48B is an assembled view of the multiple lumen access device of FIG. 48A.
FIG. 49 is an assembled view of a multiple lumen access device similar to that shown in FIG. 48B with infusion ports formed on a hub of the multi-ribbed hollow obturator.
FIG. 50A is an exploded view of a multiple lumen access device with a multi-ribbed solid obturator telescopically fitting within an introducer with infusion ports.
FIG. 50B is an assembled view of the multiple lumen access device of FIG. 50A.
FIG. 51 is an exploded sectional view of a multiple lumen access device with a multi-ribbed hollow obturator telescopically fitting within a tapered introducer with an infusion port.
FIG. 52 is an assembled view of the multiple lumen access device of FIG. 51.
FIG. 53 is a sectional view of the obturator seen in FIG. 51 taken along line 53-53.
FIG. 54 is a perspective view of a portion of the obturator seen in FIG. 51.

### DESCRIPTION OF THE PREFERRED EMBODIMENTS

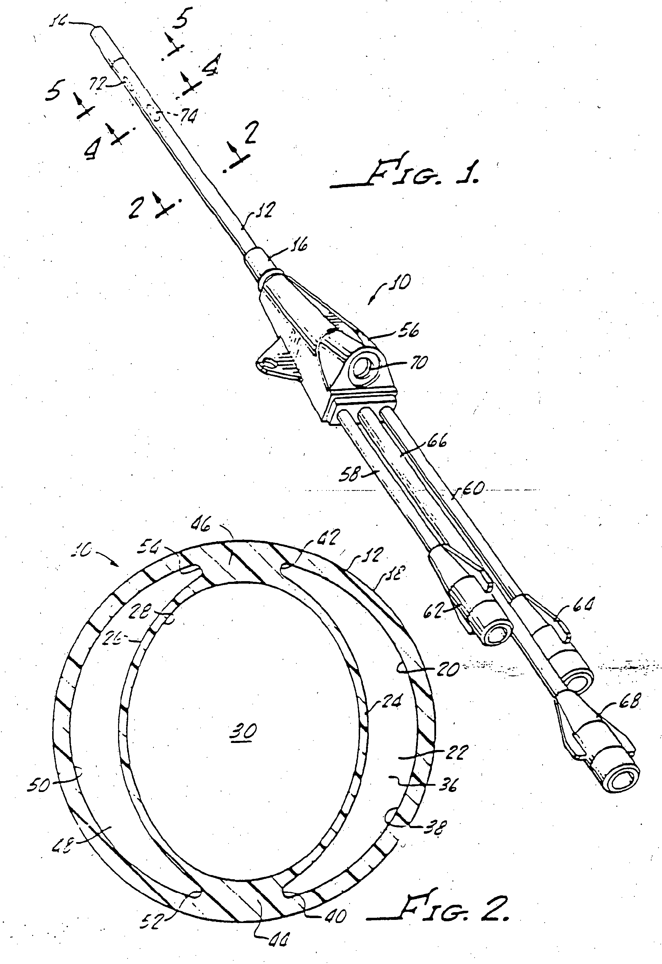

An exemplary multiple lumen access device (MLAD) is shown generally at 10 in FIGS. 1-5. The device 10 includes an outer tube 12 which has a distal end 14 and a proximal end 16. As best shown in FIGS. 2-5, the outer tube 12 has an exterior surface 18 and an interior surface 20. The interior surface 20 defines an access passageway or lumen 22 which has a cross-sectional area that may vary at different locations between the distal 14 and proximal 16 ends of the outer tube 12. Typically, the outer tube 12 may be tapered at the distal end 14, if desired. As a result of the tapering of the outer tube 12, the cross-sectional area will decrease accordingly.

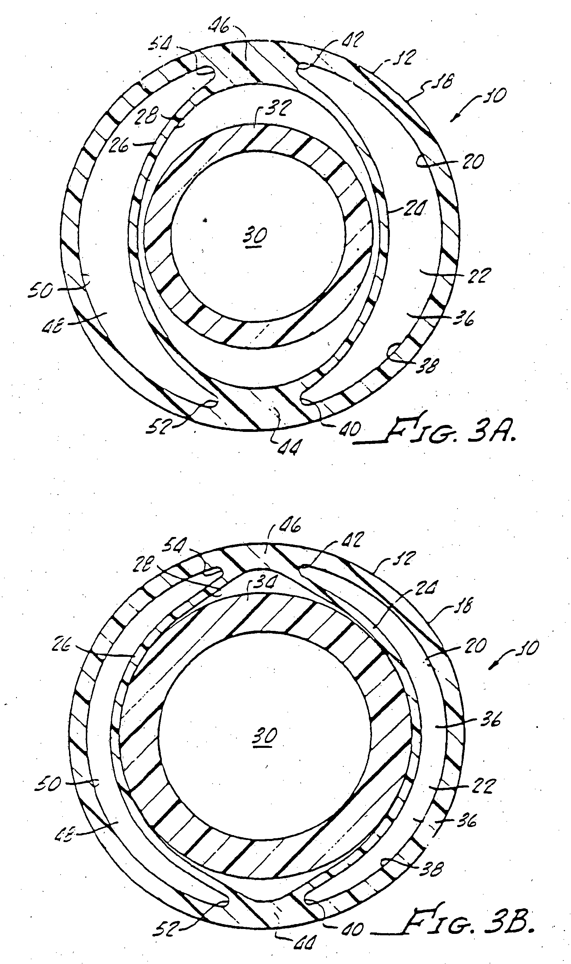

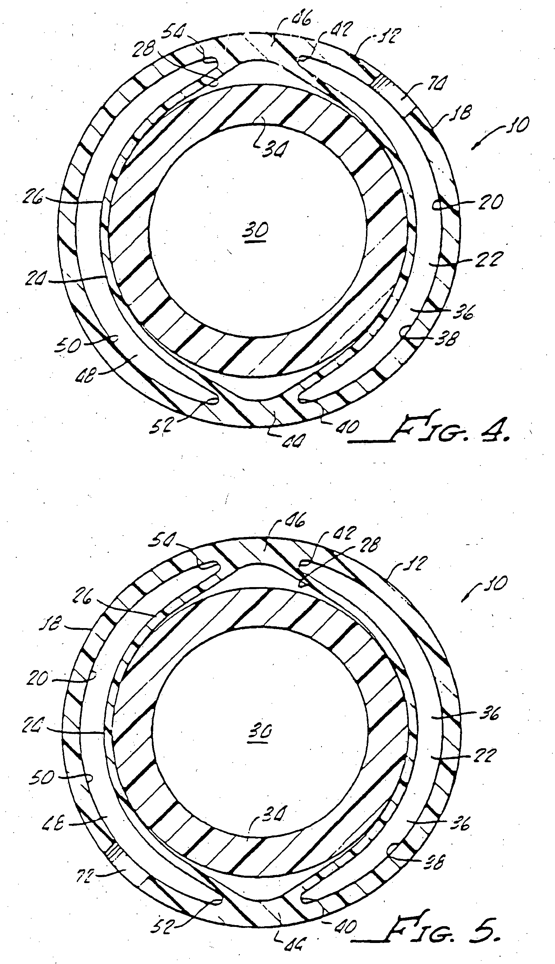

An inner tube 24 is located within the access passageway 22. The inner tube 24 has a distal end and a proximal end which correspond to the distal end 14 and proximal end 16 of the outer tube 12. As illustrated in FIG. 2, the inner tube 24 is formed by a wall surrounding a device lumen 30, the wall having an exterior surface 26 and an interior surface 28. The interior surface 28 defines a device lumen 30 through which medical implements (such as catheters 32 and 34 shown in FIGS. 3A and 3B, respectively) may be inserted into the body. Catheter 34 is also shown in position within the device lumen 30 in FIGS. 4 and 5.

Two auxiliary lumens 36 and 48 are located between the exterior surface 26 of the inner tube 24 and the interior surface 20 of the outer tube 12. The auxiliary lumens 36 and 48 each have a distal end and a proximal end which correspond generally to the distal and proximal ends of the outer tube 12 and inner tube 24. In this particular embodiment, the surfaces which define the auxiliary lumens 36 and 48 correspond to portions of the interior surface of the outer tube and exterior surface of the inner tube. Specifically, auxiliary lumen 36 is defined or bordered by an interior surface 38 which corresponds to the interior surface 20 of the outer tube 12 and the exterior surface 26 of the inner tube 24. Further, the auxiliary lumen 36 is defined by separation surfaces 40 and 42 which are formed by separation barriers 44 and 46, respectively.

A second auxiliary lumen 48 is also formed or defined by the interior surface 20 of the outer tube 12 and the exterior surface 26 of the inner tube 24. Accordingly, the interior surface 50 which defines the second auxiliary lumen 48 corresponds to these surfaces. In addition, the auxiliary lumen 48 is bordered by separation surfaces 52 and 54 formed by separation barriers 44 and 46, respectively.

Referring to FIG. 1, the multiple lumen access device 10 includes a junction housing 56. The junction housing 56 is connected to the proximal end 16 of the access lumen 12. The housing 56 includes infusion tubes 58 and 60 which are connected through the housing 56 to auxiliary lumens 36 and 48, respectively. The infusion tubes 58 and 60 include luer connectors 62 and 64. Other conventional connection devices may be used. A third infusion tube 66 is connected via the housing 56 to the device lumen 30 in order to provide a route for infusion of liquid into the device lumen 30. It should be noted that the infusion tube 66 is not connected to the junction housing 56 at a right angle as is typically done in conventional introducer-type devices. Instead, the infusion tube 66 extends from the housing 56 parallel to the other two infusion tubes 58 and 60. This parallel orientation of the tubes 58, 60 and 66 allows housing 56 to be a low profile body which reduces the bulkiness of the proximal end of the device and increases its wearing comfort. A conventional locking device, such as luer lock 68 is also provided at the proximal end of the infusnn tube 66.

The housing 56 includes a valve 70 through which various medical implements are inserted into the device lumen 30. Valve 70 includes a valve or gasket assembly which is designed to provide sealing of the device lumen 30 when medical implements are both present and absent from the device lumen 30. Any of the known gasket arrangements and valve mechanisms used to provide sealing of introducers and related medical implement access devices are suitable. The multiple lumen access device 10 is designed for use in combination with providing access to either the arterial or venous sides of the bloodstream.

An opening 72 (*see* FIG. 1 and FIG. 5) is provided towards the distal end of outer tube 12. The opening 72 is provided to allow exit of fluid from auxiliary lumen 48 which has been introduced through infusion tube 58. Likewise, an opening 74 (shown in phantom in FIG. 1 and also shown in FIG. 4) is provided for allowing the fluid introduced through infusion tube 60 to exit auxiliary lumen 36 at the proximal end of the outer tube 12.

As illustrated in FIGS. 1, 4 and 5, the openings 72 and 74 are preferably sized to avoid restricting fluid flow through the respective auxiliary lumens. Therefore, it is preferred that the openings 72 and 74 are each sized sufficiently large to be equal or greater than the maximum distended/expanded cross-sectional area of the corresponding auxiliary lumens 36 and 48. Of course, this same principle applies with regard to any number of auxiliary lumens each having a variable cross-section. When either auxiliary lumen 36, 48 is under pressure and no device is present in the device lumen 30, the auxiliary lumen cross-section increases in diameter. In one embodiment, the auxiliary lumen increases, for example, from approximately 15 gauge to about 12 gauge, while in another embodiment the auxiliary lumen increases from approximately 18 gauge to about 14 gauge. Therefore, the openings 72 and 74 are each sized to be equivalent to or greater than 12 gauge or 14 gauge, respectively, to avoid restricting fluid flow through the respective auxiliary lumen. When other cross-section diameters of the auxiliary lumens are used, the size of the openings, such as 72 and 74, are preferably sized accordingly.

In this exemplary embodiment, the inner tube 24 must be sufficiently flexible to be stretchable between a relaxed position as shown in FIG. 3A and various expanded positions as exemplified in FIG. 3B. In FIG. 3A, a catheter 32 having a diameter of 1.3 millimeter (4 French) is shown inserted within the device lumen 30. The inner tube 24 is in a relaxed position where the cross-sectional area of the device lumen 30 is approximately 2 square millimeters. The relaxed cross-sectional area of the device lumen 30 will preferably range from 1 to 3 square millimeters. Larger diameters are possible, if desired. It is preferred, but not required, that inner tube 24 have a circular or elliptical cross-section.

As shown in FIG. 3B, a larger diameter catheter 34 has been inserted into the device lumen 30. The inner wall 24 is made from sufficiently resilient material and is sufficiently sized so that it can expand to the diameter shown which is approximately 3 millimeter (9 French). The maximum diameters to which the inner tube 24 can be expanded is limited by the diameter of the outer tube 12. The inner tube 24 may be flexed inward, if desired, by applying fluid pressure through one or both auxiliary lumens 36 and 48. Typically, the cross-sectional area of the device lumen 30 when the inner tube 24 is in its maximum expanded state will range from 5 to 9 square millimeters. Larger diameters are possible, if desired. Preferably, the inner tube 24 will be sufficiently flexible so that it can be expanded completely outward against the interior surface 20 of the outer tube 12. In the fully expanded state, the auxiliary lumens 36 and 48 will have substantially reduced cross-sectional areas. However, it is preferred that the auxiliary lumens 36 and 48 not be entirely closed. It is desirable to leave some opening through these two auxiliary lumens 36 and 48 at all times to allow flushing fluids to be passed through the lumens in order to prevent the formation of blood clots or other problems associated with a completely collapsed lumen.

Preferably, the inner tube 24 is sufficiently flexible to be stretched to expanded positions wherein the cross-sectional area of the device lumen 30 in the expanded state is up to 85 percent of the cross-sectional area of the access lumen 22. This allows for continual auxiliary fluid introduction through auxiliary lumens 36 and 48. Further, it is preferred that in the relaxed position as shown in FIG. 3, that the device lumen 30 have a cross-sectional area which is not less than 35 percent of the cross-sectional area of the access lumen 22.

The inner tube 24 is preferably connected to the outer tube 12 at separation barriers 44 and 46 in order to divide the access lumen 22 into a three-chamber lumen, i.e. the central device lumen 30 and two auxiliary lumens 36 and 48. In order to achieve the desired flexibility of the device lumen 30, it is preferred that a relatively elastic material be utilized. Suitable elastic materials include, but are not limited to, polyvinylchloride, polyurethane, polyethylene, nylon, silicone, fluoropolymers and polypropylene. Further, in order to achieve the desired variation in lumen cross-sectional areas, the thickness and durometer of the inner tube walls 24 must be carefully matched to the particular material being utilized. For less flexible materials, the wall thicknesses must be correspondingly reduced in order to achieve the desired flexibility limits. The inner tube 24 should be sufficiently flexibb so that it can be expanded to diameters which are at least as large as the outer tube 12.

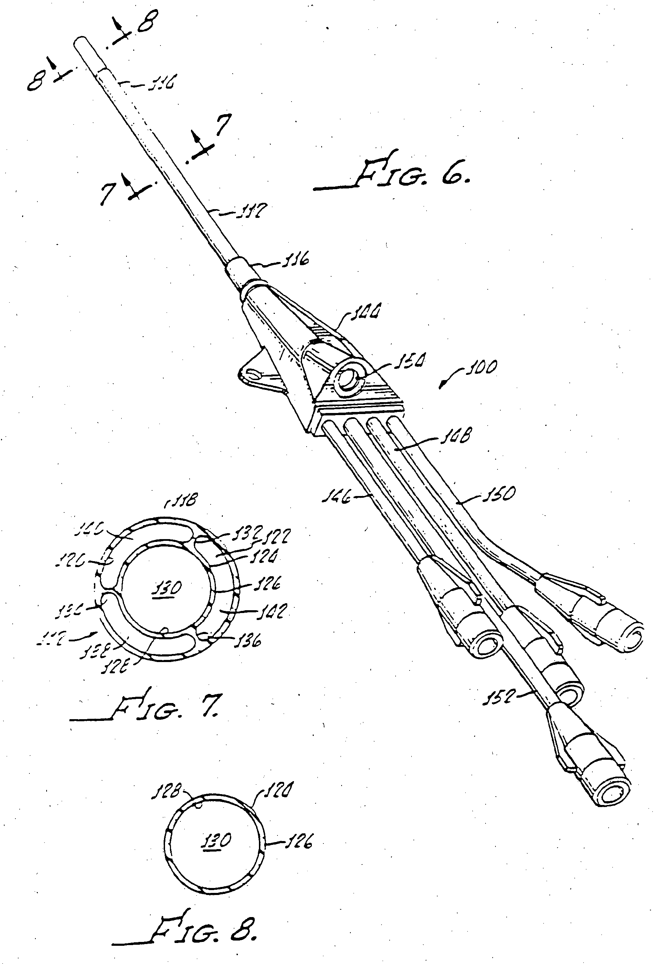

Another exemplary embodiment is shown generally at 100 in FIG. 6. The access device 100 is similar to the previous embodiments in that it includes an outer tube 112 having a distal end 114 and a proximal end 116. As best shown in FIGS. 7 and 8, the outer tube 112 has an exterior surface 118 and an interior surface 120. The interior surface defines an access passageway 122 in which an inner tube 124 is located. The inner tube 124 includes an exterior surface 126 and an interior surface 128. The interior surface 128 of the inner tube 124 defines a device lumen 130 through which medical implements, such as a catheter, may be inserted. The access device 100 includes three separation barriers 132, 134 and 136 which, in combination with the interior surface of the outer tube 120 and exterior surface of the inner tube 126, form three auxiliary lumens 138, 140 and 142. The multiple lumen access device 100 includes the same type of junction housing 144 which was described in the previously-described embodiment (FIGS. 1-5), except that an additional infusion lumen is included to provide infusion of liquid into the additional auxiliary lumen. As shown in FIG. 6, infusion lumens 146, 148 and 150 are connected via junction housing 144 to auxiliary lumens 138, 140 and 142, respectively. A primary infusion lumen 152 is also provided for introducing fluids into the device lumen 130. Again, an access port 154 is provided with the appropriate gaskets and/or valving mechanism to allow introduction of catheters and other medical devices into the device lumen 130.

The inner tube 124 in this exemplary embodiment may or may not be made from flexible material. The inclusion of three separation barriers in this particular embodiment reduces the ability for flexible expansion and contraction of the inner tube 124. However, it is preferred that the material used to form the device lumen 124 and the separation barriers be more flexible than the exterior outer tube 112 in order to allow variations in the cross-sectional areas of the auxiliary lumens. Otherwise, the same materials and fabrication techniques which are used to fabricate the prior embodiments are also suitable for use in making the multiple lumen access device 100.

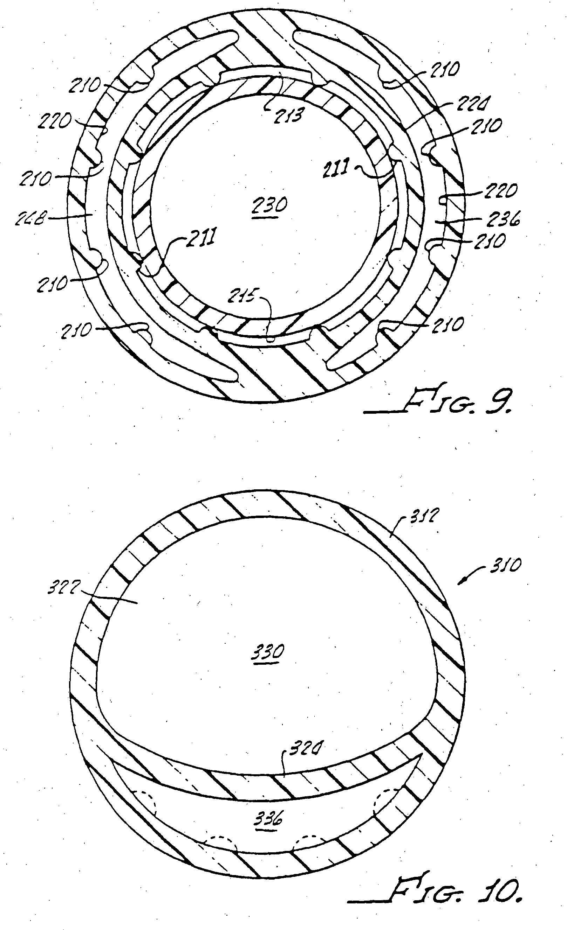

In an embodiment, as shown in FIG. 9, spacer ribs 210 are provided on the interior surface 220 of the outer tube 212 to prevent the inner tube 224 from being expanded to a position which closes the auxiliary lumens 236 and 248. Spacer ribs 211 may also be provided to insure that a passageway 213 is maintained around a device 215 when it is located within device lumen 230. The ribs 210 are preferably located longitudinally along the entire length of the outer tube 212 where the inner tube 224 is also present. The particular cross-sectional shape of the spacer ribs 210 is not particularly important so long as they are relatively blunt and do not damage the inner tube 224 during contact therewith. The number and relative positioning of the spacer must be chosen to insure that complete closure of the auxiliary lumens 236 and 248 does not occur. For inner tubes 224 which are relatively flexible, the number and size of ribs may have to be increased. The ribs 210 shown in FIG. 9 are an example of a preferred configuration. The number, shape, size and position of the ribs 210 may be varied as required in order to prevent closure of the auxiliary lumens 236 and 248 as discussed above.

Although more than two auxiliary lumens may be included into the access device, it is preferred that two lumens be utilized. The use of two lumens is a preferred design for allowing uniform expansion of the inner tube 24 between the relaxed state as shown in FIG. 3A and an expanded state as shown in FIG. 3B.

Access devices which include one auxiliary lumen are also possible. The cross-section of an exemplary access lumen is shown at 310 in FIG. 10. The access lumen 310 includes an outer tube 312 which defines an access lumen 322. The access lumen 322 is divided into a device lumen 330 and an auxiliary lumen 336 by an inner flexible wall 324. The inner surface of the outer wall 312 preferably includes spacer ribs (shown in phantom at 350) to prevent closure of the auxiliary lumen 336. The inner wall 324 is made from the same types of flexible materials as described previously for the inner tubes used in the multiple auxiliary lumen embodiments. This particular embodiment is well-suited for use in those situations where a relatively large device lumen is required in favor of the advantages provided by multiple auxiliary lumens.

The outer wall 12 is preferably made from any of the well-known polymer materials used in fabricating introducers and other access devices. Exemplary materials include polyurethane, polyethylene, polypropylene, nylon, polyester, polyether/ester copolymers, silicone based polymers, metalocene catalyzed polyolefins or ethylene vinyl acetate and synthetic rubbers. Preferably, the material used and wall thicknesses for the outer wall 12 are such that the outer wall 12 is a relatively stiff tube in relation to the inner tube 24. Further, the material used for the outer wall 12 should be compatible for molding purposes with the material used to form the inner wall 24. It is preferred that the outer wall 12 and inner wall 24 be extruded together, as will be more fully described below. The outer wall 12 and inner wall 26 may be made from the same material or different materials. The inner wall 26 is preferably made from softer versions of the various polymers listed above. When using different materials, the materials must be compatible for bonding or fusing together.

Other fabrication techniques for connecting the inner and outer tubes are possible provided that the connection between the two lumens at the separation barriers 44 and 46 extends the entire length of the two lumens and provides a solid integral connection between the lumens. For example, radio frequency (RF) welding of the tubes is another possible fabrication procedure which may be used to make the access lumen. If desired, the entire triple lumen can be extruded as a single integral multiple lumen structure.

During use, the exemplary access device 10 allows introduction of medical implements into the device lumen while at the same time allowing infusion of fluid through tube 66 also into device lumen, as well as allowing infusion through tubes 58 and 60 into auxiliary lumens 48 and 36, respectively. Since, as discussed above, the outer tube 12 is relatively inflexible in the radial direction (though overall longitudinally flexible), the total available cross-sectional area for insertion of medical implements and flow of fluids is limited for a given access device. However, the flexibility of the device lumen allows the doctor or other medical professional to selectively and fully utilize the total available cross-sectional area.

In FIG. 3A, a relatively small catheter 32 is shown inserted within the device lumen 30. In this configuration, fluids may be infused/removed through the unused area of the device lumen 30 as well as the two auxiliary lumens 36 and 48. It should be noted that the preferred design inherently centers the catheter or medical implement 32 so that the auxiliary lumens 36 and 48 have approximately equal cross-sectional areas. However, it should be noted that the application of differential pressure to the infusion tubes 58 and 60 can be used to selectively increase or decrease the relative cross sectional areas available for infusion of fluids through the auxiliary lumens. For example, the size of auxiliary lumen 36 can be increased relative to the cross-sectional size of auxiliary lumen 48 by introducing the infusion of liquid through tube 58 at a pressure which is relatively higher than that of tube 60. The double auxiliary lumen design of this exemplary embodiment is especially well suited for providing such differential fluid flows when desired.

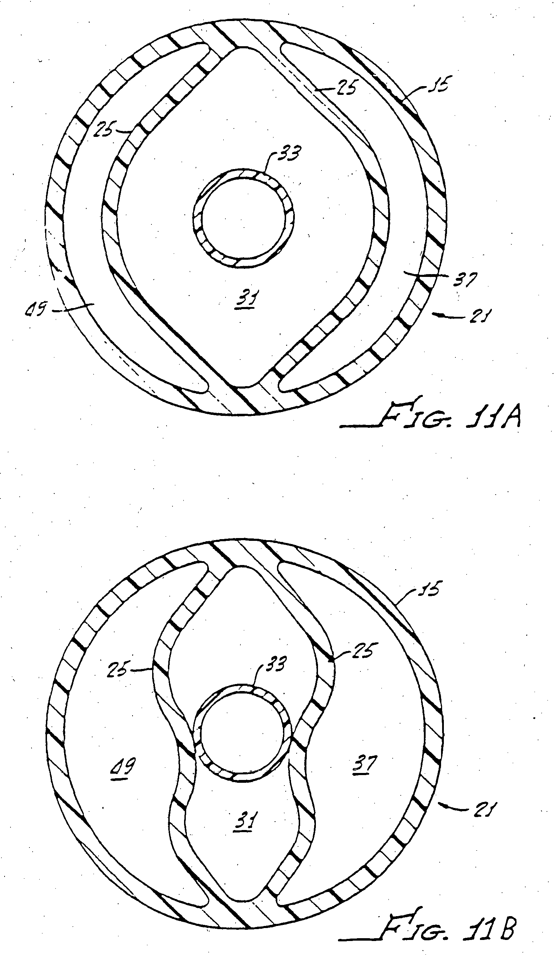

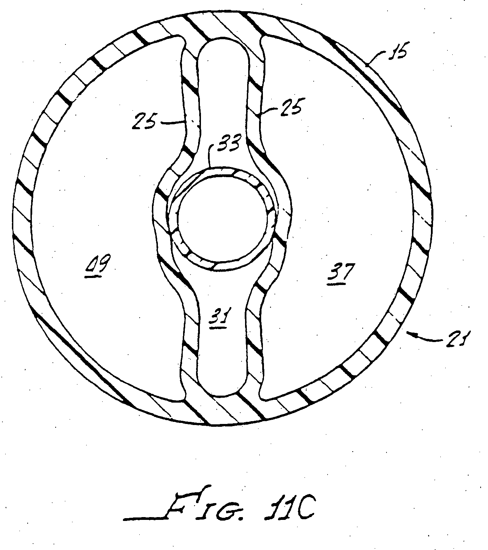

An exemplary embodiment which further demonstrates the flexibility of devices is demonstrated in FIGS. 11A-11C. In FIG. 11A, an exemplary access device 21 is shown in which a relativdy small catheter 33 is located within the device lumen 31. In this configuration, fluids may be infused/removed through the unused area of device lumen 31 as well as the two auxiliary lumens 37 and 49. As shown in Fig. 11A, the inner flexible walls 25 is in a relaxed position. In this position, the inner wall 25 is relatively close to the outer wall 15. When desired, the size of the auxiliary lumens 37 and 49 can be increased substantially by increasing the pressure of liquids being passed therethrough. The result, as shown in Fig. 11B, is the partial collapsing of the inner tube or inner walls 25 about the catheter 33. In the partially contracted or collapsed position as shown in Fig. 11B, the inner walls 25 are not stretched. Instead, their configuration changes as shown in FIG. 11B to accommodate the change in relative sizes of the auxiliary lumens and device lumen. As shown in FIG. 11C, the size of auxiliary lumens 37 and 49 are increased even further to a point where the fluid flow through the two auxiliary lumens is maximized. In this condition, stretching of the contracted flexible walls 25 may occur. As is apparent from FIGS. 11A-11C, it is possible to provide a wide variance in fluid flows through the auxiliary lumens and device lumen depending upon differential pressures applied through the various lumens.

### Alternative Sheath Cross-Sections

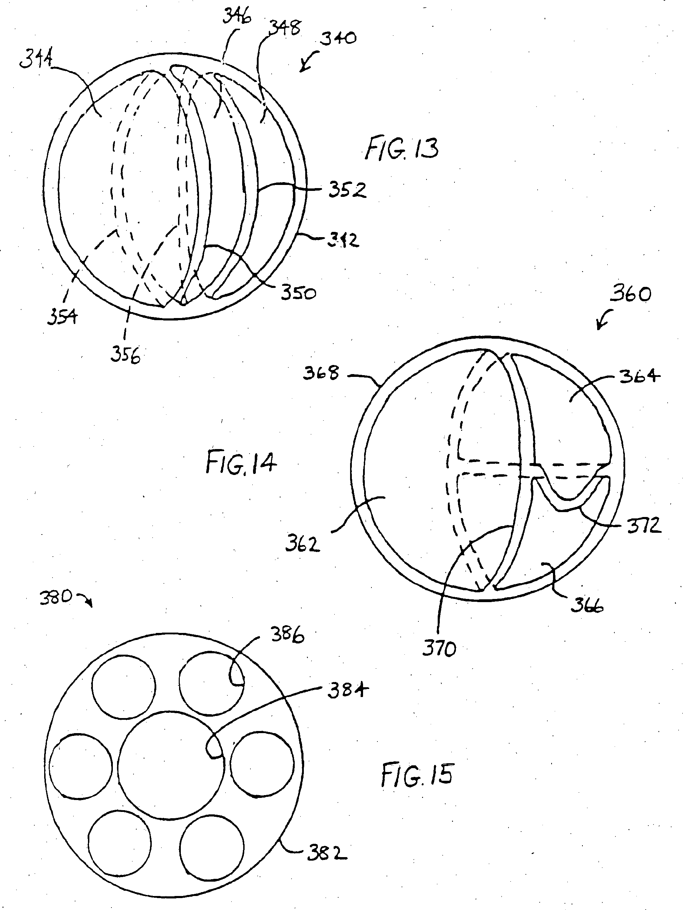

Figure 13 illustrates an alternative cross-section of a sheath portion 340 for a multiple lumen access device in which the device lumen is not between two auxiliary lumens. The sheath portion of the devices comprise the portion that is distally disposed with respect to the junction housing, defines multiple lumens therein, and is substantially inserted into the patient's vasculature. In FIG. 13, the sheath portion 340 comprises an outer tube 342 defining within, and, in series from left to right, a device lumen 344, a first auxiliary lumen 346, and a second auxiliary lumen 348. A first flexible wall 350 separates the device lumen 344 from the first auxiliary lumen 346, while a second wall 352, that can be flexible or relatively rigid, separates the first and second auxiliary lumens 346, 348. The first flexible wall 350 can move from its position shown in solid line to the dashed-line position shown at 354 as the pressure difference across the wall increases in favor of the first auxiliary lumen 346. Likewise, the second flexible wall 352, if flexible, can move from its position shown in solid line to the dashed-line position shown at 356 as the pressure difference across the wall increases in favor of the second auxiliary lumen 348.

Figure 14 is a further alternative cross-section of a sheath portion 360 for a multiple lumen access device. The embodiment of Figure 14 is similar to that shown in Figure 13, and includes a device lumen 362, first auxiliary lumen 364, and second auxiliary lumen 366, all defined with an outer tube 368. In contrast to the embodiment of Figure 13, the auxiliary lumens 364 and 366 are not arranged side-by-side, but are instead stacked on top of one another (at least in the orientation shown) so that both are located adjacent the device lumen 362. In this respect, a generally T-shaped internal dividing wall is provided including an elongated wall portion 370 and a shorter wall portion 372. The shorter wall portion 372 separates the first and second auxiliary lumens 364,366, while the elongated wall portion 370 separates the two auxiliary lumens from the device lumen 362. Both the elongated wall portion 370 and the shorter wall portion 372 are curvilinear in their relaxed configurations, shown in solid line in Figure 14. The wall portions 370 and 372 straighten out into the dashed-line positions upon an increase in pressure in one or both of the auxiliary lumens 364, 366 relative to the device lumen 362.

In another alternative embodiment, not illustrated, the device lumen can be provided between two or more auxiliary lumens of different sizes. The device lumen is typically positioned off-center between crescent-shaped auxiliary lumens, and at least one of the auxiliary lumens can be expandable in accordance with the preceding discussion (that is, a wall between one of the auxiliary lumens and the device lumen is flexible). Desirably, there are two auxiliary lumens and the larger of the two lumens is expandable to enable infusion of large flow rates. In one particular embodiment, the larger lumen has a capacity equivalent to a gravity flow through a 14 gauge lumen.

Figure 15 illustrates a still further cross-sectional view of a sheath portion 380 which may be used in conjunction with a multiple lumen access device. In this embodiment, the sheath portion 380 includes a generally cylindrical solid member 382 having a central device lumen 384 and a plurality of auxiliary lumens 386 surrounding the device lumen formed therein. There are no flexible walls in this embodiment, it being understood that various aspects may be advantageously utilized without the need for varying the cross-sectional shape of any of the lumens within the sheath portion 380. Alternatively, if desired, any wall portion separating the device lumen 384 from any of the auxiliary lumens 386 may be formed to be flexible to enable variability of the cross-section of that lumen.

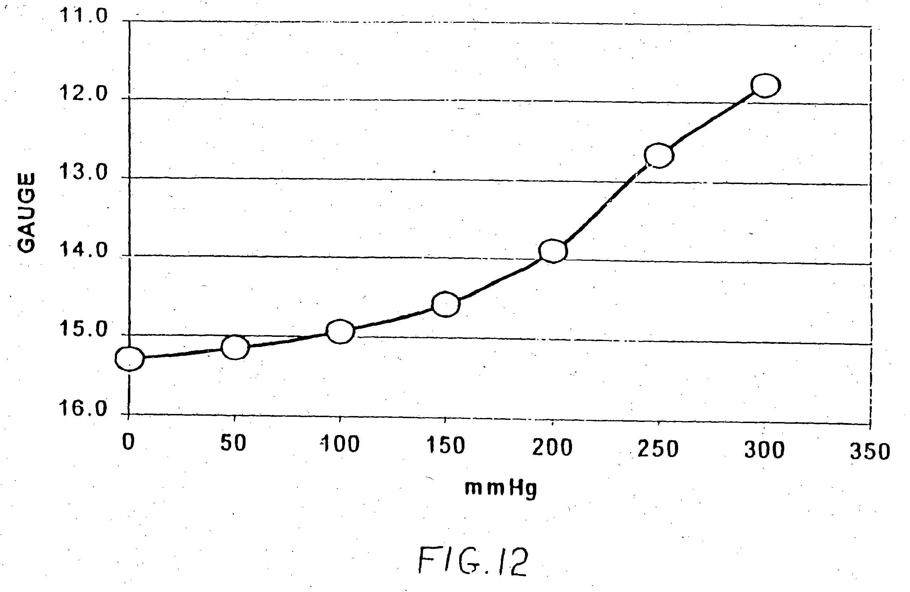

The graph illustrated in FIG. 12 shows that as pressure inside the auxiliary lumen increases the cross-sectional area of that lumen increases. (The convention is that cross-section in terms of "gauge" numbers actually decreases for larger areas). FIG. 12 reflects the pressure response of one exemplary mutli-lumen catheter wherein the auxiliary lumen increases in size from about 15 gauge when there is no flow therethrough, to about 12 gauge with fluid infusion at a pressure of about 300 mmHg (in this sense, the 300 mmHg is the differential pressure across the flexible wall, if the assumption is made that the device lumen is at atmospheric pressure). The response curve of the increase in lumen size indicates that the flexible wall is sufficiently rigid to withstand small changes in pressure. From 0-150 mmHg, the auxiliary lumen increases only from slightly smaller than 15 gauge to slightly larger than 15 gauge. Only above 150 mmHg pressure differential does the lumen size significantly increase. This response is a factor of the thickness, shape and material of the flexible wall between the device and auxiliary lumens.

One of the advantages of having an inner wall 25 (as seen in FIG. 11A) or inner wall 350 (as seen in FIG. 13) which is flexible but also sufficiently rigid is that a pressure transducer may be connected to the multi lumen access device to monitor a central venous pressure of a patient. In particular, the pressure transducer (not shown) may be placed in communication with one of the auxiliary lumens 37 and 49 to measure the central venous pressure. Advantageously, the resistance to small pressure differentials described above enables more accurate pressure monitoring, because the flexible wall does not substantially flex upon small differentials in pressure, and thus does not dampen or attenuate the resultant pressure wave sensed externally to the lumen. Specifically, the flexible inner walls 25 have sufficient stiffness to avoid significant damping or attenuation of pressure pulses in the auxiliary lumens 37 and 49, and do not undergo major flexing from small pressure differentials as shown in FIG. 12.

As described previously in regards to the exemplary embodiment illustrated in FIGS. 1-5, the outer wall 15 of the embodiment illustrated in FIGS. 11A-11C is preferably made from any of the well-known polymer materials used in fabricating introducers and other access devices. Preferably, the material used and wall thickness for the outer wall 15 are such that the outer wall 15 is a relatively stiff tube in relation to the inner walls 25 in the radial direction. Further, the material used for the outer wall 15 should be compatible for molding purposes with the material used to form the inner walls 25. It is preferred that the entire cross-section of the multi-lumen portion of the device 10, including the outer tube 12 and inner walls 25, is extruded together from a homogeneous material. Alternatively, the outer wall 15 and inner walls 25 may be coextruded and that the junctions 27 be formed by molding of the inner 25 and outer wall 15 together during the coextrusion process. Therefore, outer wall 15 and inner walls 25 may be made from the same material or different materials. The inner wall 25 is preferably made from softer versions of the various polymers listed previously. When using different materials, the materials should be compatible for bonding or fusing together.

The above described exemplary embodiments may be used in the same manner as conventional introducer devices. Additionally, if desired, the devices may be used in the same manner as conventional central venous pressure catheters. As will be appreciated by those skilled in the art, the multiple lumen access device provides the design flexibility to allow use as a single device where the capabilities of an introducer device and catheter are simultaneously required. For example, many diagnostic and invasive medical procedures require the insertion of guide wires and/or medical devices, while simultaneously monitoring critical bodily functions and introducing or removing fluids as needed. The access device allows all of the above functions to be performed simultaneously and selectively through a single access device.

### MLAD WITH VALVE INSERT

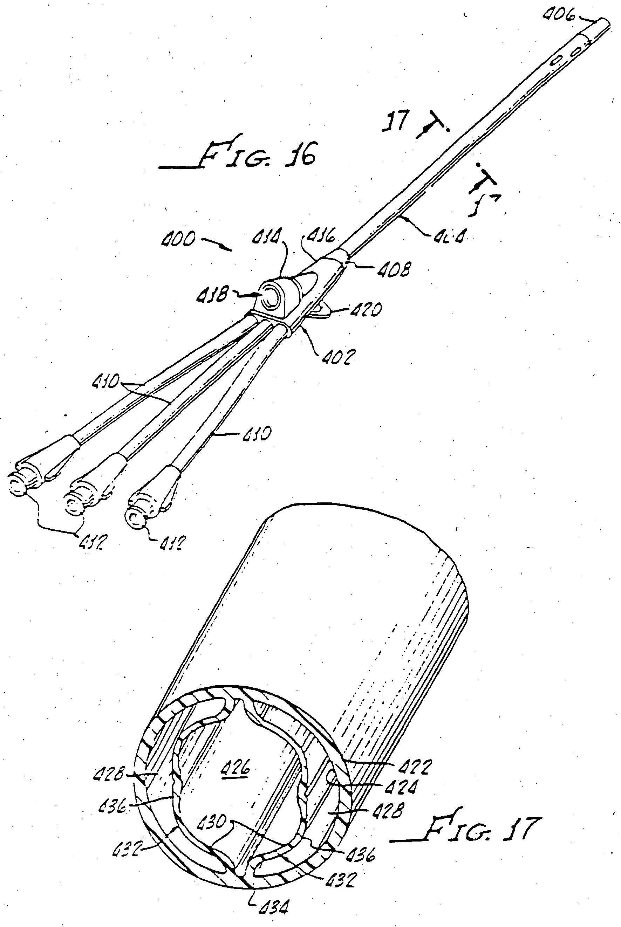

FIG. 16 illustrates an alternative multiple lumen device 400 (MLAD) with an improved junction housing 402. The device 400 is similar to the FIGS. 1-5, and includes a multiple lumen sheath 404 extending distally from the housing 402. The multiple lumen sheath has a distal end 406 for insertion in a body cavity and a proximal end 408 attached to the housing 402. A plurality of extension tubes 410 is attached to the proximal end of the housing 402 and terminate in luer connectors 412. The housing comprises a valve insert portion 414 and a low profile lumen portion 416. A valve insert 418 is secured in a cavity defined in the portion 414. A pair of mounting wings 420 is integrally formed with the junction housing 402 for attaching to a patient.

The multiple lumen sheath 404 seen in cross-section in FIG. 17 comprises an outer circular tube 422 having an interior surface 424. In the illustrated embodiment, the multiple lumen sheath 404 includes a central device lumen 426 and a pair of auxiliary lumens 428 disposed on opposite sides of the device lumen. The device lumen 426 is defined between interior surfaces 430 of a pair of divider walls 432. The divider walls extend in a non-linear fashion substantially across the entire outer tube 422 and terminate at junctions 434. The junctions 434 are spaced a slight distance from one another so that the sheath 404 does not exhibit the separation barriers, as previously described. As illustrated, the device lumen 426 is generally concentrically positioned within the outer tube 422 and has a nominal diameter of slightly greater than half the outer tube 422. Between exterior surfaces 436 of the divider walls 432 and the interior surfaces 424 of the outer tube 422, the auxiliary lumens 428 are formed. The lumens 428 are substantially crescent shaped and are shown identical in size. Of course, as described previously, various other lumen configurations can be provided in the multiple lumen sheath 404.

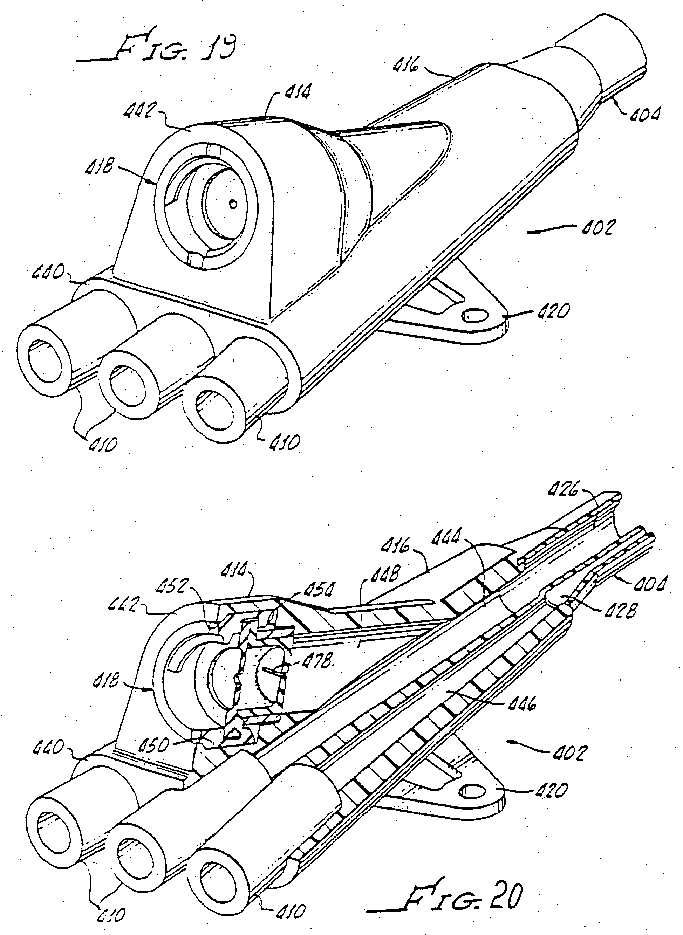

The junction housing 402 is illustrated in greater detail in FIGS. 19 and 20. The low profile lumen portion 416 has an oval cross-section tapering gradually wider along its long axis from the multiple lumen sheath 404 to a proximal face 440 to which the extension tubes 410 connect. The valve housing portion 414 angles upward from one wide surface of the lumen portion 416 and terminates in a proximal face 442. The device access valve insert 418 fits within an angled cavity formed in the valve housing portion 414. With specific reference to FIG. 19, the lumen portion 416 comprises a main channel 444 and a pair of auxiliary channels 446 on either side. The main charnel communicates with a central extension tube 410, while the auxiliary channels 446 communicate with the side extension tubes. A device channel 448 defined within the valve housing portion 414 is in communication with the main channel 444 and angles upwardly therefrom to terminate in a widened cavity 450. The cavity 450 receives the valve insert 418 which is held therein by a circumferential lip 452 on the outermost portion of the cavity 450. The cavity 450 continues inward from the lip 452 towards the device channel 448 and narrows at a step 454. The step 454 provides a stop surface against which the valve insert 418 is pressed. Desirably, the insert 418 and cavity 450 are keyed to facilitate insertion in a particular rotational orientation and prevent further rotation.

### VALVE INSERT

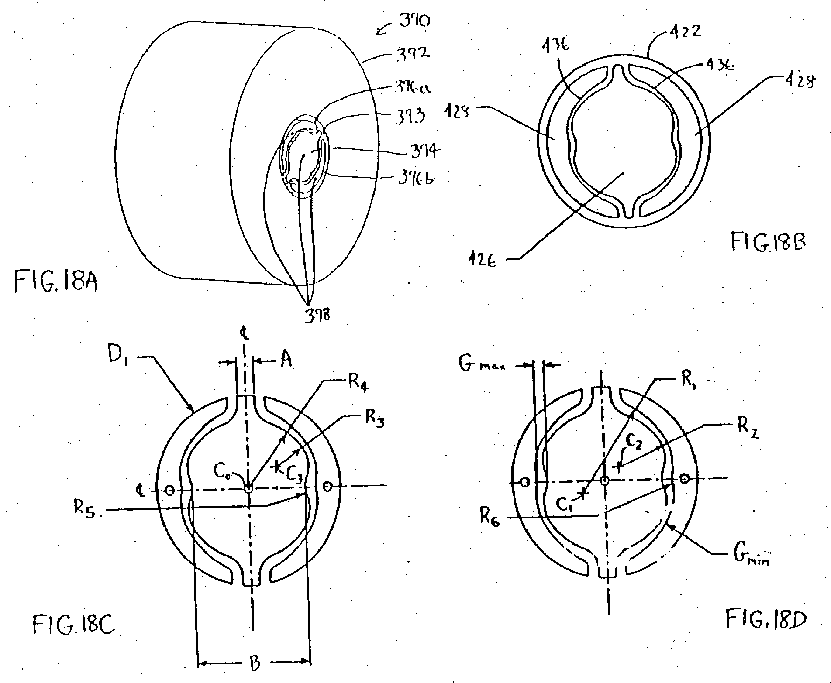

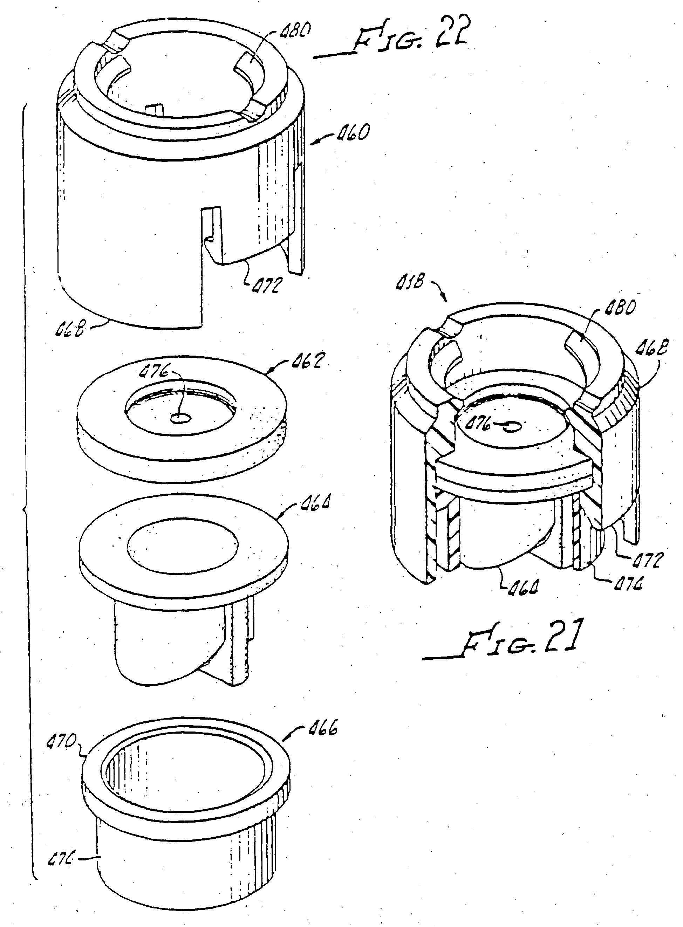

Now with reference to FIGS. 21 and 22, the device access valve insert 418 is seen in greater detail. The valve insert 418 comprises four components: an outer frame 460, a wiper 462, a valve 464, and a sleeve 466. The assembled valve insert 418 is seen in FIG. 18. The wiper 462 and valve 464 are juxtaposed within an outer wall 468 of the frame 460, and held therein by the interaction between a flange 470 of the sleeve 466 and a pair of cantilevered latches 472 provided on the frame. The sleeve 466 further includes a support tube 474 projecting downward from the flange 470 and surrounding the valve 464. The wiper 462 includes an aperture 476 through which device catheters may be inserted in a sealed fashion. The valve 464 may be a conventional duck-billed valve having a valve slit 478, as seen in FIG. 17. The combination of the wiper 462 and the valve 464 effectively seals the device channel 448 formed within the junction housing 402 and the exterior of the junction housing when devices are repeatedly introduced and withdrawn through the valve insert 418. The outer wall 468 further includes a pair of partial threads 480 which cooperate with exterior threads on an infusion catheter dilator or contamination shield (not shown).

The entire valve insert 418 is formed separately from the junction housing 402, which is molded from a soft, flexible material, typically a soft thermoplastic material. The softness of the junction housing 402 is important in enhancing patient comfort and flexibility of the entire multi-lumen access device 400 when assembling and mounting to a patient. Conversely, the frame 460 of the valve insert 418 is relatively rigid for supporting the wiper 462 and duck-billed valve 464. The wiper and duck-billed valve are made of elastomeric materials, and the outer wall 468 prevents valve depression or distortion and thus enhances the patency of the seal formed by the valve insert 418. The sleeve 466 stabilizes the elastomeric valve components, and the support tube 474 provides an outer surface against which the duck-billed valve 464 cannot extend past. The rigidity of the valve insert 418 provides structure to facilitate connection of devices thereto. Furthermore, the junction housing 402 is easily injection molded over the multiple lumen sheath 404 and tubes 410 prior to addition of the insert 418, for a simplified manufacturing process.

### SHEATH CROSS-SECTION FORMATION AND DETAILS

Figure 18A illustrates in perspective an extrusion die 390 used to extrude a preferred cross-section of sheath portion of a multiple lumen access device, such as the cross-section shown in Figure 17. The extrusion die 390 comprises a large tubular member 392 having a bore 393, and a plurality of lumen-forming mandrels positioned longitudinally therein. Specifically, a device lumen-forming mandrel 394 and two surrounding auxiliary lumen-forming mandrels 396a, 396b are positioned within the bore 393 using elongated pins (not shown) closely fitting within guide holes 398.

As it is known in the extrusion art, material such as polyurethane in liquid form can be forced through the cavities formed between the bore 393 and the mandrels 394,396 and gradually cooled so that when the material exits from the extrusion cavity it has solidified somewhat and retains the shape shown in Figure 18B.

Figure 18B is a cross-sectional view of the exemplary sheath 404 of Figure 16 and includes an outer tube 422 and two inner walls 436 together defining device lumen 426 and the surrounding auxiliary lumens 428, as described in more detail below.

Figures 18C and 18D are more detailed views of the surfaces of the mandrels 394 and 396 in one embodiment. The outer diameter of the auxiliary lumen-forming mandrels 396 is given as D₁, and the outer surfaces are centered about axis C₀. The inner surfaces of the mandrels 396 are defined by several arcs. As seen in Figure 18D, a first inner surface portion as a radius R₁ centered about axis C₁, while second portion has radius R₂ centered about axis C₂.

The device lumen-forming mandrel 394 includes two diametrically opposed ribs 398 having a thickness A, and a central non-uniform convex body defined by several arcs that generally conform to the inner surfaces of the auxiliary lumen-forming mandrels 396. More specifically, the exemplary mandrel 394 includes convex surfaces that are identical in the four quadrants shown and have a first radius R₃ centered about axis C₃, and a second radius R₄ centered about axis C₀. A minimum gap indicated at Gₘᵢₙ is defined between the convex outer surfaces of the device lumen-forming mandrel 394, and the concave inner surfaces of the auxiliary lumen-forming mandrels 396. The minimum gap Gₘᵢₙ thus forms the thinnest portions of the walls 436 of the device.

Along the diametric plane that is normal to the diametric plane through the ribs 398, both extrusion mandrels exhibit a curvature toward the axis C₀ Namely, the device lumen-forming mandrel 394 has a concave outer surface portions with the radius R₅, and both of the auxiliary lumen-forming mandrels 396 have a convex portion with a radius R₆. The configuration of these curvilinear portions creates a maximum gap between the mandrels indicated at Gₘₐₓ. The maximum gap Gₘₐₓ thus forms the thickest portions of the walls 436. The walls 436 are initially spaced apart a distance B.

Exemplary dimensions of the extrusion die and the corresponding cross-section of the device sheath are given in the table below:

**TABLE I -- Extrusion Mandrel Configuration**

| DIMENSION | VALUE (in, mm) |
|---|---|
| D₁ | 0.325, 8.26 |
| R₁ | 0.172, 4.37 |
| R₂ | 0.0956, 2.43 |
| R₃ | 0.0574, 1.46 |
| R₄ | 0.1195, 3.04 |
| R₅ | 0.0382, 0.97 |
| R₆ | 0.0201, 0.51 |
| A | 0.0306, 0.78 |
| B | 0.2007, 5.10 |
| Gₘᵢₙ | 0.0099, 0.25 |
| Gₘₐₓ | 0.0182, 0.46 |

The dimensions shown in Table 1 are strictly exemplary, and the multiple-lumen access device by no means is limited to these particular dimensions.

The resultant cross-section of the sheath after extrusion through the die 390 is seen in both FIGS. 17 and 18B. The two walls 436 each connect to the outer tube 422 at closely-spaced locations that are approximately diametrically opposed. The walls 436 bow away from one another in their relaxed states, with each generally following the curvature of the outer tube 422 to form therebetween the auxiliary lumens 428. The device lumen 426 is formed between the walls 436 which are well-suited to collapsing upon a positive pressure gradient generated between an auxiliary lumen 428 and the device lumen. That is, the narrow gaps Gₘᵢₙ formed in the extrusion die create regions in each wall 436 that are weak in bending. As the pressure differential across the walls 436 increases in favor of the auxiliary lumen 428, the thickest portion created by the gap Gₘₐₓ tends to be forced inward first because of the bending of the thinnest portions. If a device is positioned within the device lumen 426, the walls 436 will contact it at the thickest portions first. This behavior is shown for a different sheath cross-section in FIGS. 11A-11C. As a result, the line contact between the walls 436 and the device facilitates sliding movement of the device through the sheath. That is, the walls 436 bend such that a large surface area is prevented from contacting the device, and thus the frictional resistance to sliding movement is minimized.

### ALTERNATIVE MLAD WITH VALVE INSERT

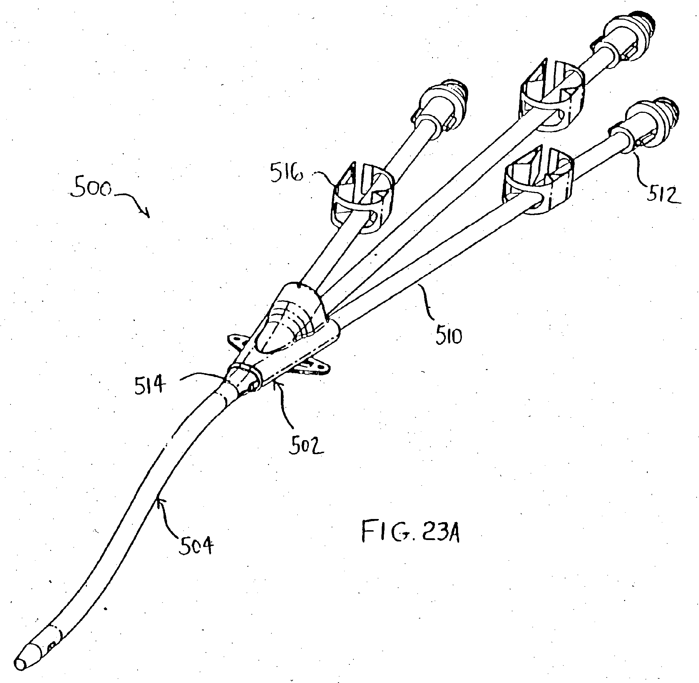

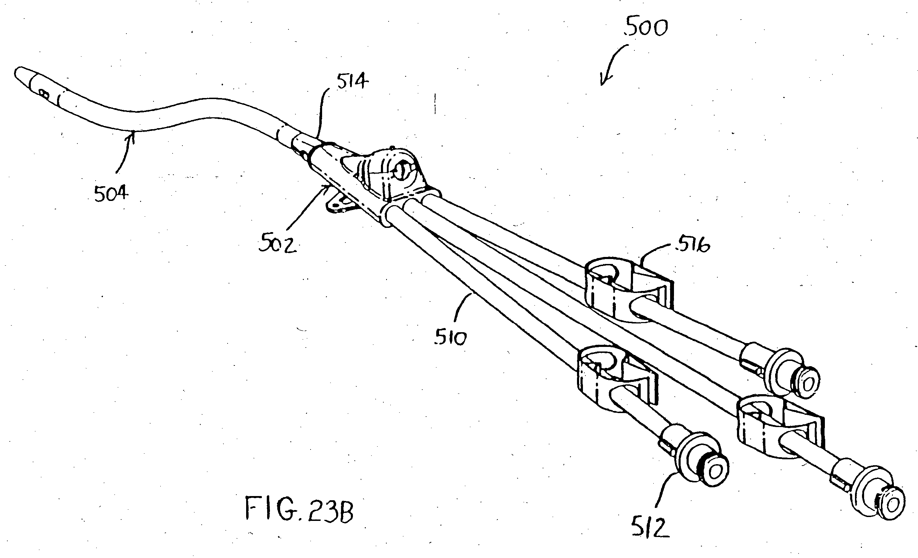

Figures 23A and 23B are different perspective angles of an exemplary multiple lumen access device 500, which is in many respects very similar to the device 400 shown in Figure 16. The device 500 includes a junction housing 502, a distal sheath 504, and a plurality of proximal extension tubes 510 terminating in luer connectors 512. One of the main distinctions from the earlier described embodiment is the provision of a strain relief insert 514 positioned at the distal end of the junction housing 502. In addition, an alternative device valve insert is provided, but is not seen in Figures 23A and 23B and will be described in detail below. Finally, a plurality of conventional finger-actuated clamps 516 are mounted on the extension tubes 510.

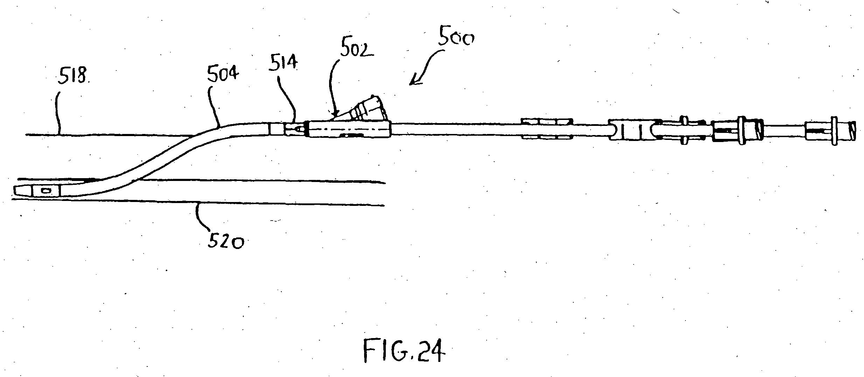

Figure 24 is a side elevational view of the device 500 of Figure 23 showing the distal sheath 504 inserted through the outer tissue 518 of a patient and into a vessel 520. The flexible nature of the sheath 504 is seen in this figure, as well as the ability of the junction housing 502 to live flat against the patient's skin. As mentioned above, the material used and wall thicknesses for the outer tube of the sheath 504 are such that the outer tube is a relatively stiff tube in relation to the inner flexible walls. Nevertheless, the entire sheath 504 is sufficiently pliable so as to enable slight bending along its length which facilitates insertion into the patient's vessel and comfortable placement against the skin. The soft material used in making the junction housing 502 further prevents irritation to the patient. In addition, the strain relief insert 514 is located adjacent the most extreme bend of the sheath portion 504 and helps prevent kinking of the internal lumens.

### ALTERNATIVE VALVE INSERT

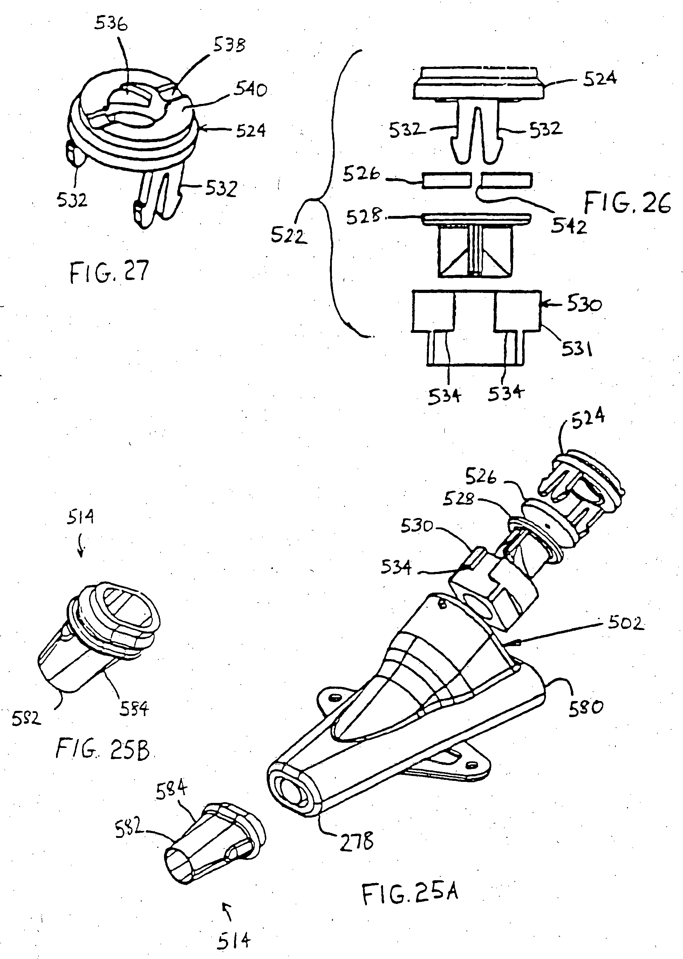

Figure 25A is a perspective view of the junction housing 502 with the strain release insert 514 exploded from the distal and, and components of an alternative device lumen valve insert 522 exploded from the proximal end. The strain relief insert 514 is additionally shown at a different angle in Figure 25B. Figures 26 and 27 illustrate the components of the alternative valve insert 522 in greater detail.

FIGS. 26 and 27 illustrate the alternative device access valve insert 522 which includes a tactile feedback feature. The valve insert 522 comprises four components: a clamp 524, wiper 526, valve 528, and lower outer frame 530. The wiper 526 and valve 528 are juxtaposed within an outer wall 531 of the lower outer frame 530, and held therein by the securement of the clamp 524 onto the lower outer frame 530 by a pair of latches 532 which engage with mating lugs 534. The clamp 524 includes a pair of partial threads 536 which cooperate with exterior threads of an infusion catheter dilator or contamination shield (not shown). A pair of grooves 538 is disposed on a contact face 540 of clamp 524. The wiper 526 includes an aperture 542 through which device catheters may be inserted in a sealed fashion. The valve 528 may be a conventional duck-billed valve having a valve slit, as seen at 464 in FIG. 22. As described previously in regards to the device valve insert 418 shown in FIGS. 21 and 22, the combination of the wiper 526 and the valve 528 effectively seals the device channel formed within the junction housing and the exterior of the junction housing when devices are repeatedly introduced and withdrawn through the valve insert 522.

The upper portion of the valve insert 522 is relatively rigid and may be formed from the same material as the lower outer frame 530 such as acrylic, polysulfone, or other high durometer materials. It is also noted that the valve insert 522 shown in FIG. 26 may be used for the exemplary multi-lumen access devices shown in FIGS. 1, 6 and 16.

### CONTAMINATION SHIELD ADAPTER

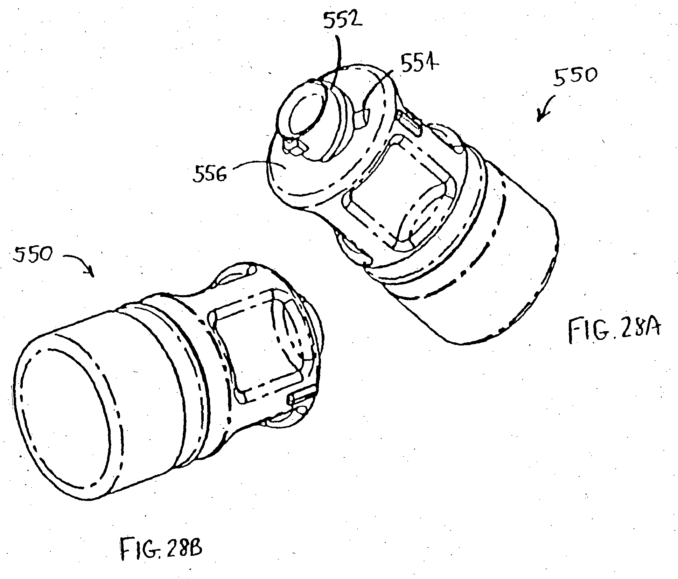

FIGS. 28A and 28B illustrate an adapter 550 for a distal end of a contamination shield. The adapter 550 includes threads 552 which mate with the threads 536 of the upper portion of the valve insert 532 illustrated in FIGS 26 and 27. The threads 552 of the adapter 550 are designed to fully engage with the threads 536 of the clamp 524 by a 1/4 turn of the adapter 550. A pair of lugs 554 are disposed on the contacting surface 556 of the adapter 550 such that the lugs 554 mate with the pair of grooves 538 of the clamp 524. As the 1/4 turn is completed, the lugs 554 snap into the grooves 538 and create a tactile feedback. The contamination shield 550 sealingly receives a flexible tubular sheath thereover to provide a sterile channel that is alternately collapsible and extensible around devices inserted through the device valve. Such contamination shields are well known in the art and will not be further described.

### STRAIN RELIEF INSERT

A multiple lumen access device may kink at the multi-lumen sheath/junction housing interface when the access device is attached to a patient. The kink may reduce the cross-sectional area of the multi-lumen sheath or in extreme circumstances, result in blockage of the lumens. The "kink" problem may be resolved by providing a multiple lumen access device with the strain relief insert 514 as illustrated in FIGS. 23A, 23B, 24, and 25A. Again, the access device 500 is similar to the access device described in FIG. 16 with the exception that the junction housing 502 is modified to accept the strain relief insert 514. The strain relief insert 514 is connected to the distal end of the junction housing 502, and over the multi-lumen sheath 504.

The strain relief insert 514 has an oval cross-section tapering gradually wider along its long axis from the multi-lumen sheath 504 to the junction housing 502. As seen in FIG. 25A, the low profile lumen portion 578 of the junction housing 502 also has an oval cross-section tapering gradually wider along its long axis from the strain relief insert 514 to a proximal face 580 to which the extension tubes (not shown) connect. The strain relief insert 514 includes a tapered body 582 having ribs 584 which gradually blend into the body. These ribs 584 allow the strain relief insert 514 to flex and prevent the multiple-lumen sheath 504 from kinking.

In order to achieve the desired flexibility of the strain relief insert 514, it is preferred that a relatively soft, elastic material be utilized. Suitable elastic materials include, but are not limited to, polyurethane and pellathane with a 55D shore hardness. Further, in order to achieve the desired flexibility, the thickness of the strain relief insert 514 must be carefully matched to the particular material being utilized. For less flexible materials, the wall thickness should be correspondingly reduced in order to achieve the desired flexibility limits. The strain relief insert 514 may be formed using radio frequency (RF) technology with appropriate forming dies and fixtures. Desirably, the strain relief insert 514 is overmolded onto the sheath 504 and subsequently coupled to the junction housing 502 at the time that the housing and sheath are connected.

### MLADS WITH REMOTE INTRODUCER VALVES

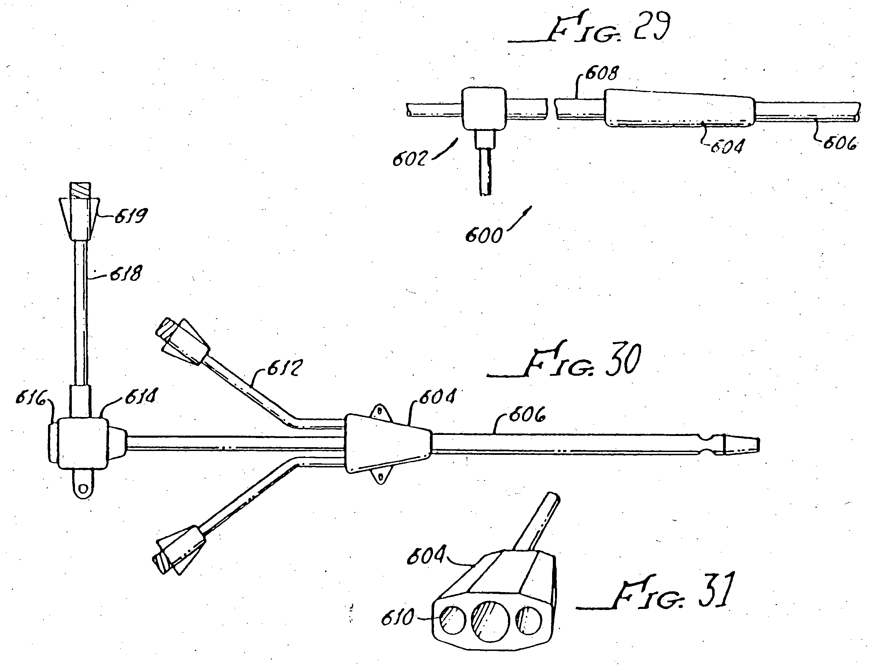

FIGS. 29 and 30 illustrate a further embodiment of the multiple lumen access device 600 in which the device access valve 602 is not formed integrally with the junction housing 604. More particularly, as best seen in FIG. 29, the junction housing 604 has a low profile which is slightly greater than the sheath 606 or extension tubes 608 attached thereto. FIG. 31 shows a proximal end of low profile junction housing 604 illustrating three channels 610 formed therein for communication with three extension tubes 612, seen in FIG. 30. A central extension tube 612 connects with a remote introducer valve 614 which has a proximal opening 616 for device catheter access. Within the introducer valve 614, a number of different duck-bill or other valves may be provided to seal the lumen of the extension tube 612 from the exterior. Introducer valve 614 may include a side port extension tube 618 terminating in a luer lumen hub 619 for attaching to infusion fluid sources. Thus, in this alternative configuration, a single needle stick followed by implantation of the multi-lumen sheath 606 is all that is required to obtain the benefits of both an introducer valve and central venous catheter, as described previously. Alternatively, the multiple lumen access device 600 further includes an auxiliary lumen valve connected to at least one other extension tube 612 than the central tube to therefore provide a valved entry to at least one of the auxiliary lumens within the sheath 606 as well as with the device lumen.

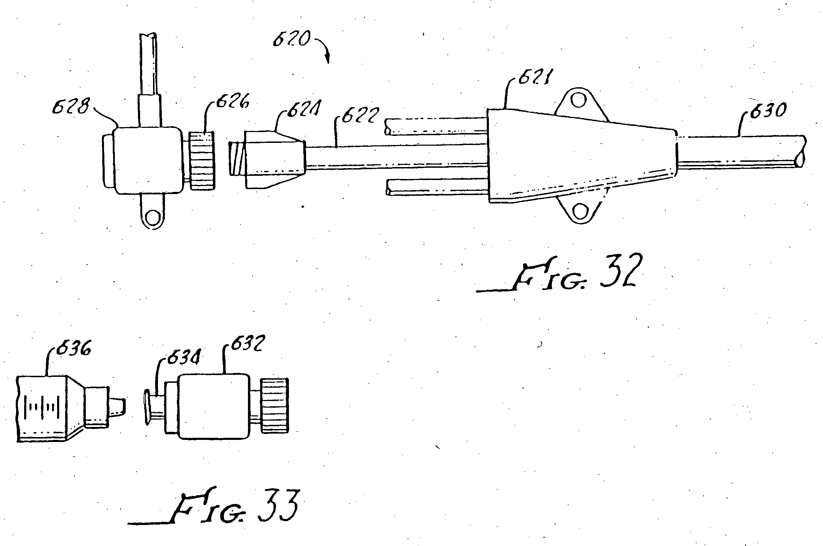

In a further alternative of the device 600, FIG. 32 illustrates a multiple lumen access device 620 wherein the central extension tube 622 terminates in a luer connector 624. The luer connector 624 is desirably used to mate with a female luer connector 626 of an introducer valve assembly 628. However, in this detachable configuration, various other medical devices having conventional luer fittings may be attached to the luer connector 624 and placed in communication with a central lumen of the multi-lumen sheath 630. FIG. 33 illustrates a further alternative, wherein the introducer valve assembly 632 is provided with a male luer connector 634 on a proximal end to which an infusion syringe 636 may be attached. As can be seen, various configurations are possible with the remote introducer valve assembly 628, and the low profile junction housing 621 is easily molded over the extension tubes and has a reduced size, thus faciltating the manufacturing process.

### MLAD WITH MULTI-LUMEN CATHETER AND/OR INTRODUCER COMBINATION

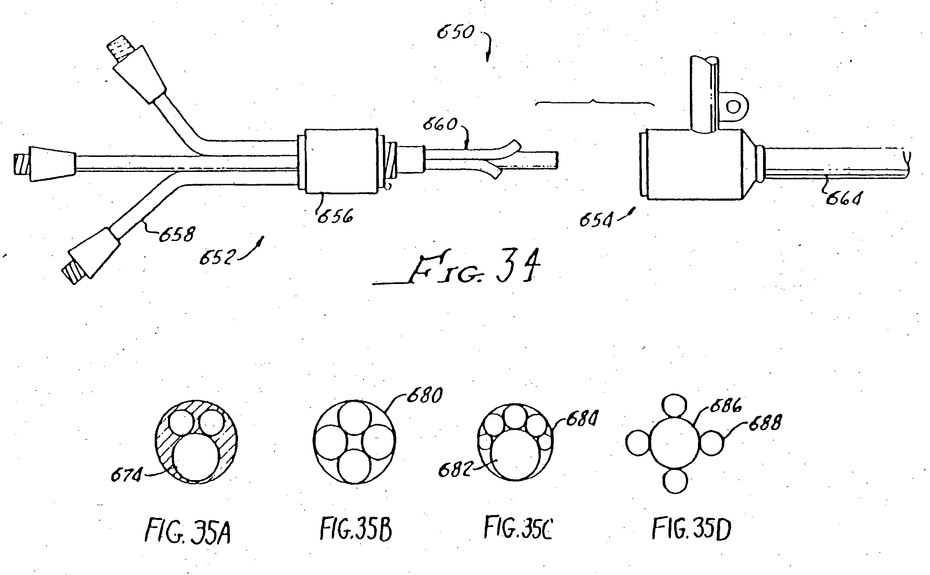

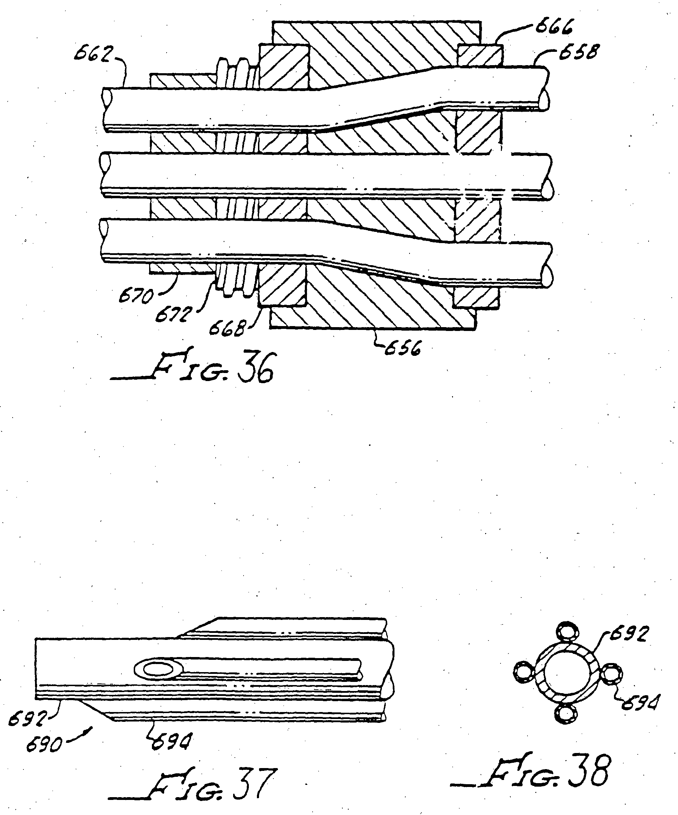

FIG. 34 illustrates a further alternative multiple lumen access device 650 comprising a multi-lumen infusion catheter 652 in combination with a conventional single-lumen introducer valve 654. The multi-lumen infusion catheter 652 includes a junction housing 656 which interfaces a plurality of proximal extension tubes 658 and a multi-lumen sheath 660 extending distally therefrom. FIG. 36 illustrates one way in which the proximal extension tubes 658 can be routed to communicate with a plurality of tubes 662 providing lumens of the multi-lumen sheath 660. The multi-lumen sheath 660 is sized to fit through the introducer valve 654 having a distal sheath 664, and from there into the body. In this manner, a single-lumen introducer may be implanted into the patient and then used further as an access port for the multi-lumen infusion catheter 652. By leaving the introducer in place, only a single stick is necessary to enjoy both introducer and central venous catheter capabilities.

With specific reference to FIG. 36, a proximal insert 666, and a distal insert 668 are mounted around the array of extension tubes 658, and distal tubes 662, respectively. The housing 656 is then formed by injection molding material around and between the inserts 666 and 668. A valve seal expander 670 may be provided to help keep the duck-bill valve within the introducer valve 654 open. Further, locking threads 672 are preferably provided to interface with the introducer valve housing 654.

FIGS. 35A-D show various configurations of the multi-lumen sheath 660. In FIG. 35A, a three-lumen solid configuration having a larger high-pressure lumen 674 is shown. FIG. 35B illustrates a four-lumen embodiment which has an outer sheath 680 so that fluid may be passed between the sheath and the exterior of the four tubes within. FIG. 35C is similar to the four-lumen sheath of FIG. 35B, but includes a single large lumen 682 and a plurality of smaller lumens 684. Finally, FIG. 35D illustrates an arrangement of lumens having a central high volume high-pressure lumen 686, and a plurality of smaller lumens 688 attached around the circumference in an even array.

FIGS. 37 and 38 illustrate a further embodiment of a multi-lumen sheath 690 having a central, high-pressure tube 692 and a plurality of outer or auxiliary tubes 694.

### MLAD WITH MULTIPLE DISCRETE TUBES

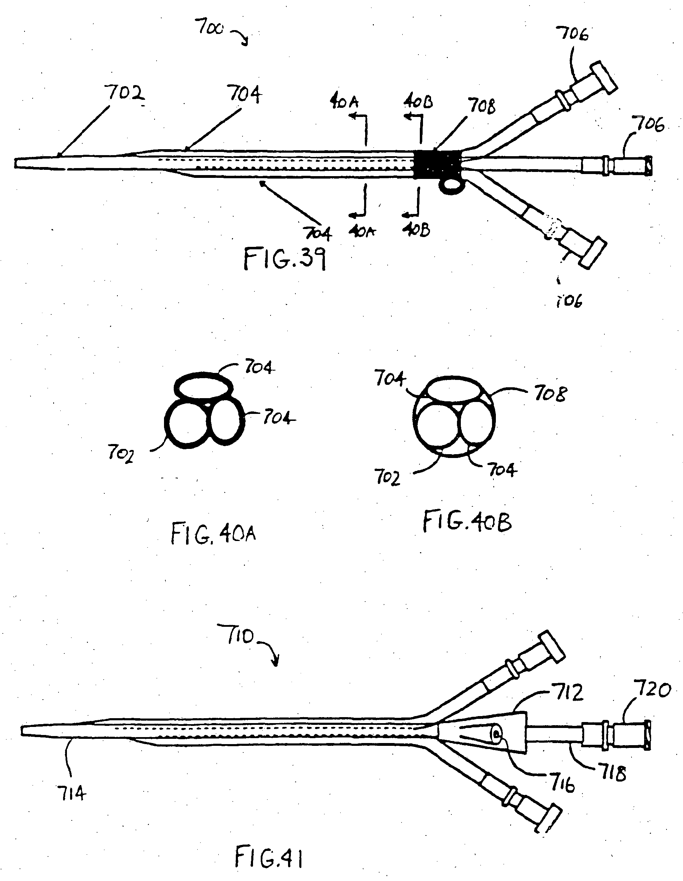

FIG. 39 illustrates a multi-lumen catheter device 700 having at least two discrete catheter tubes. In this embodiment, the multi-lumen catheter device 700 includes a main (or center) lumen tube 702 and two side lumen tubes 704. The lumen tubes 702 and 704 are configured in a side-by-side fashion, and proximal portions of the tubes 702, 704 are peeled apart to create sidearms. Hubs 706 may be attached to proximal ends of each lumen tube 702, 704 for fluid delivery or introduction of a medical device. Remote introducer valves may be connected to one or all the lumen tubes. Indeed, the device valves may be provided on any or all of the extension tubes for the various embodiments described herein and shown in any of the figures, including FIGS. 1, 6, 23A, 30. The catheter device 700 may further include a sleeve 708 at the region where the lumen tubes 702 and 704 branch outwardly. FIGS. 40A and 40B illustrate the different cross-sections of the device 700, the circular shape of the sleeve providing a smooth transition for sealing through a puncture wound into the skin. One of the advantages of this embodiment is that one or more of the lumen tubes 702 and 704 may be peeled off the multi-lumen catheter 700 if desired.

FIG. 41 illustrates another alternative multi-lumen catheter device 710. This catheter device 710 is similar to the catheter devioe 700 illustrated in FIG. 39 and includes the additional feature of a junction housing 712 connected to a proximal end of a main lumen tube 714. The junction housing 712 receives a valve insert 716 and an extension tube 718 with a hub 720 connected to its proximal end. Again, the separate tubes can be peeled away to create various lumen devices.

### MULTIPLE LUMEN CATHETER THROUGH INTRODUCER

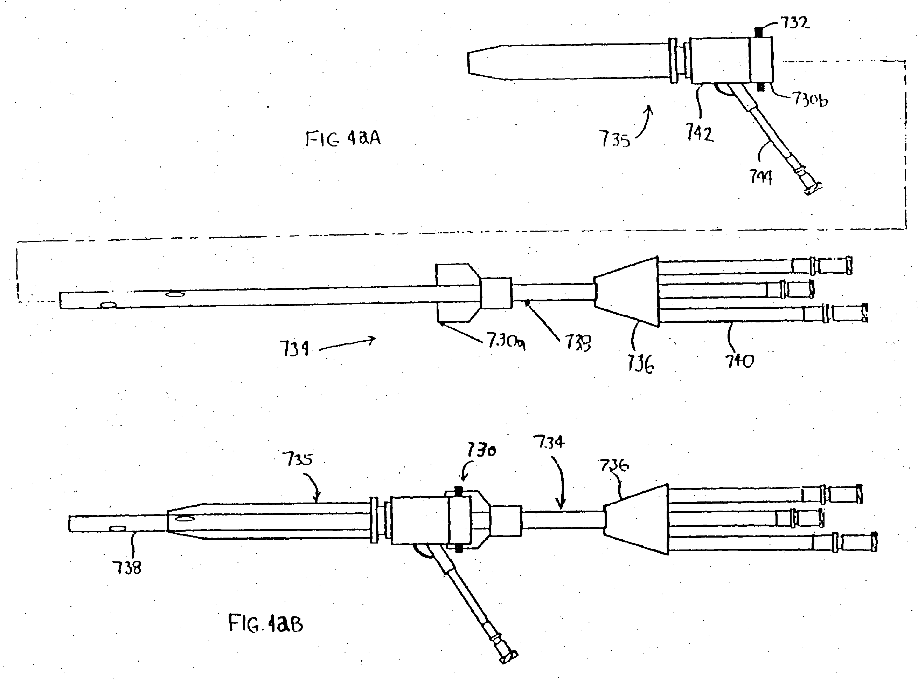

FIGS. 42A and 42B illustrate a multi-function adapter 730 for connecting different components, for example, catheters and introducers. The multi-function adapter include a first unit and a second unit that are complementary and enable a quick-release connection of a multiple lumen device and an introducer. By way of example and not limitation, the multi-function adapter may include a female unit 730a and a male unit 730b. The male unit 730b includes at least one lug 732 extending radially outward, while the female unit 730a includes a slot (not illustrated) which accepts and interlocks with the lug. The slot may be a variety of configurations to securely interlock the male unit with the female unit, such as an L-shaped channel, a bayonet lock, an interference fit, etc. Other types of adapters known in the art such as luers may be utilized as long as components of the access device can be easily connected/disconnected.

In the embodiment of FIGS. 42A and 42B, the adapter 730 couples a multiple lumen catheter 734 with an introducer 735. The catheter 734 may be a CCO catheter or other multiple-lumen device, and includes a junction housing 736 between a distal multi-lumen sheath 738 and a plurality of proximal extension tubes 740. The introducer 735 includes a hub 742 with a side arm 744 for introducing or withdrawing fluids. The female unit 730a is adapted to fit over the sheath 738 by a press fit, adhesive, or any other means generally known in the art. Conversely, the male unit may be fixedly attached to the sheath 73 8 or distal end of the junction housing 736 instead of the female unit, if desired. The adapter 730 permits detachability of the multiple lumen catheter 734 from the introducer 735 and provides great flexibility in surgical or critical care situations.

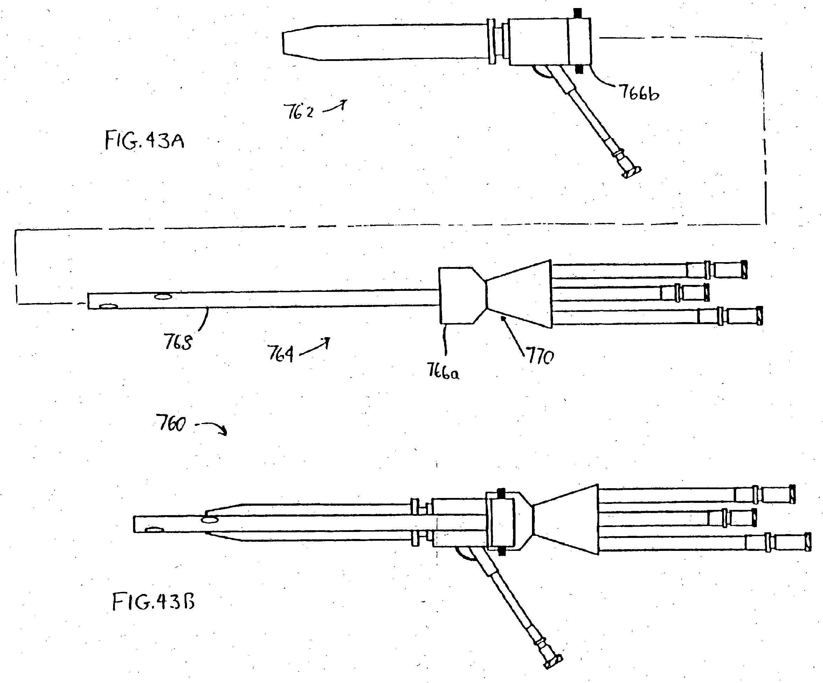

FIGS. 43A and 43B illustrate a multiple-lumen access device 760 very similar to the device of FIGS. 42A and 42B but with the adapter formed as part of a multiple lumen catheter junction housing. The access device 760 includes an introducer 762 connected to a Central Venous Catheter (CVC) or other multiple lumen catheter 764 by a multi-function adapter 766a and 766b. The catheter 764 includes a multiple-lumen sheath 768 connected to a junction housing 770.

The access device 760 (and the device of FIGS. 42) offers a significant advantage over current catheter designs in terms of cost saving and manner in which the access device 760 may be utilized. Currently, an introducer is inserted into a vein, and a surgical procedure is performed. After the surgical procedure, the introducer is usually removed and a new catheter is inserted in the vein through a second puncture and sutured onto the skin. The patient is then transported to a recovery room. By using the access device 760 illustrated in FIG. 43, the procedure can be greatly simplified. The introducer 762 is first positioned in the vessel using traditional methods, such as the Seldinger technique. After the introducer 762 is used for sampling or infusing fluids, multiple lumen catheter 764 is inserted and utilized. The catheter 764 can then be detached from the introducer 762 and removed from the vessel while the introducer 762 is left in the vessel, and the introducer 762 now functions as a catheter. Thus, after the surgical procedure, the introducer 762 does not have to be removed from the vessel and a new catheter does not have to be inserted through a second puncture.

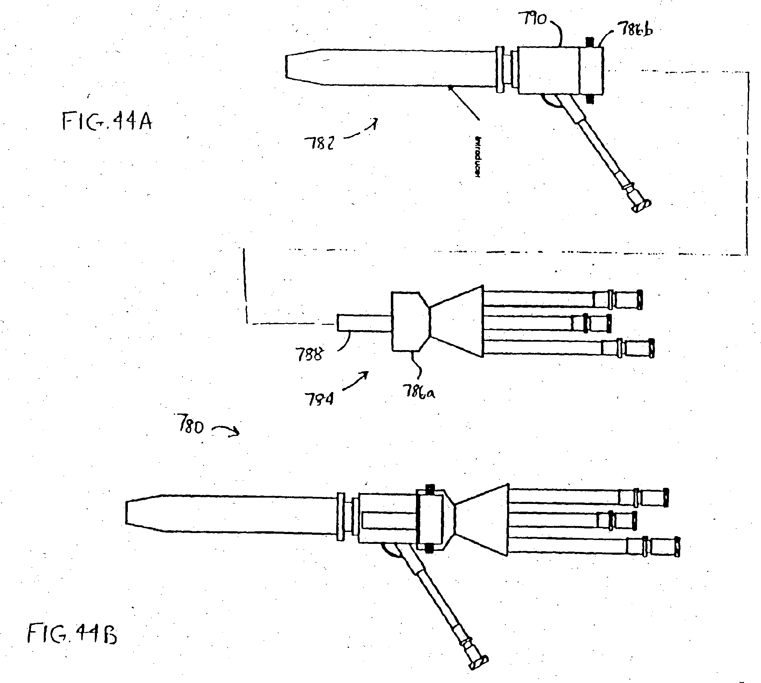

FIGS. 44A and 44B illustrate a multiple lumen access device 780 having an introducer 782 connected to a triple lumen junction housing 784 by a multi-function adapter 786a and 786b. Instead of the elongated sheath as in the previous two embodiments, the junction housing 784 includes a short hollow obturator 788 that serves to hold open a hemostasis valve in a hub 790 of the introducer 782. The three lumens within the junction housing 784 communicate with the lumen of the obturator 788 to deliver fluids to the introducer lumen.

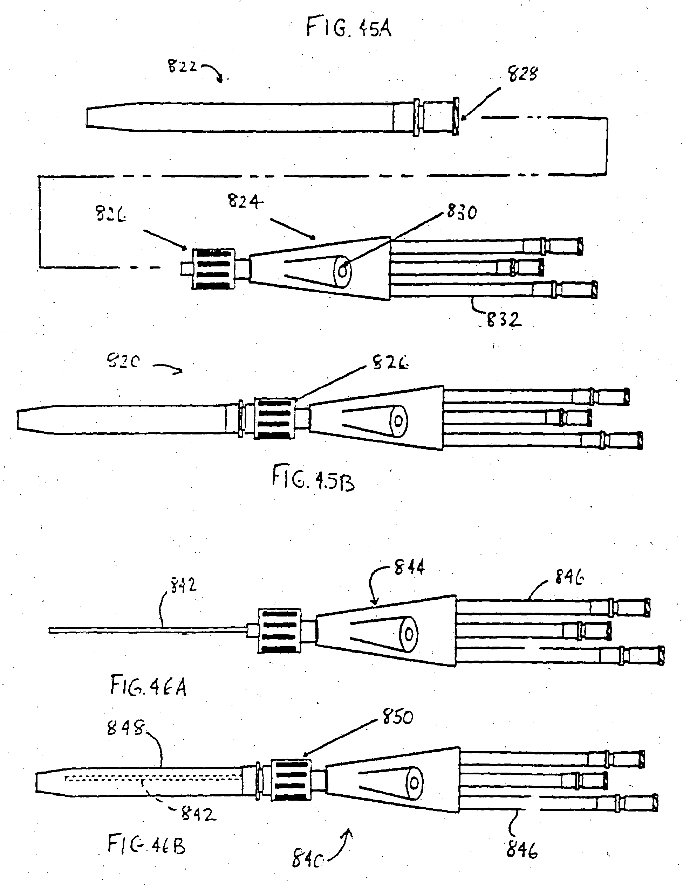

FIGS. 45A and 45B illustrate an access device 820 having a single lumen introducer 822 connected to a multiple lumen junction housing 824 by a threaded female adapter 826 and male luer connection 828. A device valve 830 in the junction housing 824 permits insertion of various devices into a vessel via the introducer 822 at the same time that various fluids are infused through extension tubes 832.

FIGS. 46A and 46B illustrate an access device 840 similar to the access device 820 illustrated in FIG. 45 and includes the additional feature of a small diameter catheter tube 842 extending from a distal end of a junction housing 844. The catheter tube 842 functions as an infusion lumen for one of the extension tubes 846, while the space between the catheter tube 842 and a single lumen introducer 848 functions as a device lumen. Again, the junction housing 844 is attached to the introducer 848 with a threaded adapter 850.

### INTRODUCER WITHIN INTRODUCER COMBINATION

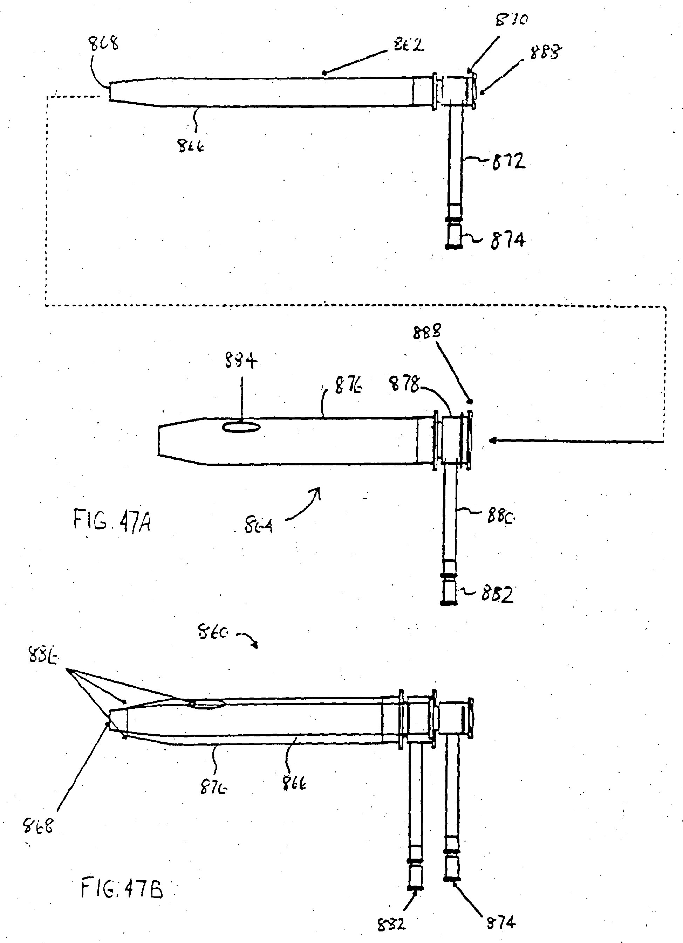

A multiple lumen access to the body through a single patient entrance site may also be accomplished by using a plurality of elongated sheaths and implements, such as introducers, obturators or catheters, inserted coaxially within each other to form multiple indcpcndent lumens. FIGS. 47A and 47B, for example, illustrate a multi-lumen access device 860 in accordance with the present invention comprising a first single-lumen introducer 862 telescopically received within a second single-lumen introducer 864. The first introducer 862 includes a single lumen sheath 866 having an opening 868 at its distal end and connected to an introducer valve housing 870 at its proximal end. Within the introducer valve housing, a duck-billed valve or other appropriate valves may be provided to seal the lumen from the exterior. The introducer valve housing 870 may include a side port extension tube 872 terminating in a hub 874 for attaching to infusion fluid sources. The second elongated implement, for example, an introducer 864 includes a single lumen sheath 876 connected to the distal end of an introducer valve housing 878. The introducer valve housing 878 also may include a side port extension tube 880 terminating in a hub 882 for attaching to infusion fluid sources, and the sheath 876 may include an opening 884 towards a distal end thereof to allow exit of fluid which has been introduced through the side port extension tube 880.

As shown in Fig. 47B, the sheath 866 of the first introducer 862 is sisd to fit coaxially through the introducer valve 878 and lumen of the second introducer 864. The distal opening 868 of the first introducer sheath 866 may extend beyond the distal end of the second introducer sheath 876. In addition, at least one of the lumens formed by the placement of introducer 862 coaxially within the introducer 864 is capable of passing a supplemental catheter. By way of example and not limitation, one such catheter has an outside diameter sized about 4 French or more. In one exemplary application of Fig. 47B, fluid 1 (for example, medicine 1) may be introduced through the hub 882 and may exit the device through the opening 884 while fluid 2 (for example, medicine 2) may be introduced through the hub 874 and exit the device through the opening 868. Alternatively, the fit between the smaller sheath 866 and larger sheath 876 may be somewhat loose at the distal end so that fluid introduced through hub 882 may pass through an annular space formed therebetween, and through the opening 884, as indicated by the arrows 886. Both introducers 862 and 864 include male luer connectors 888 on their proximal ends for connecting to a variety of medical implements, including the threaded adapters for attaching multiple lumen catheters as previously described.

The access device 860 offers a significant advantage over known introducers by providing multiple lumen access with only a single patient entrance site. Currently, two introducers are usually inserted into the patient at two different sites if another independent lumen is required. The access device 860 of the present invention allows the flexibility to start a procedure with only one introducer 864, and if another independent lumen is required, an additional introducer 862 can be inserted into the introducer 864. It is noted that the access device is not limited to two introducers. For example, a combination of three or more introducers may be coaxially configured if additional independent lumens are required.

Also, as will be understood by those skilled in the art, at least one of the single lumen introducers that is coaxially inserted into another single lumen introducer may be made from a flexible deformable material. As a result, the wall forming the sheath of such insertable introducer will also form at least one of the multiple lumens and will be movable upon differential changes in pressure across the wall. This follows from the principles described earlier with respect to extruded multiple lumen sheaths, including the descriptions related to FIGS. 3A-B, 11A-C, 12 and 17. For instance, the larger introducer sheath 876 may be rigid, while the smaller introducer sheath 866 may be flexible or pliable. If a large amount of fluid is infused through larger introducer hub 882, the spacearound the smaller sheath 866 experiences an increase in pressure and the sheath may buckle inward to accommodate the larger flow. In one embodiment, a portion of the inside introducer may be rigid and some portion may be flexible, for example only the distal tip of the smaller introducer is rigid to permit insertion through the larger introducer.

### MLADS FORMED WITH OBTURATORS WITHIN INTRODUCERS

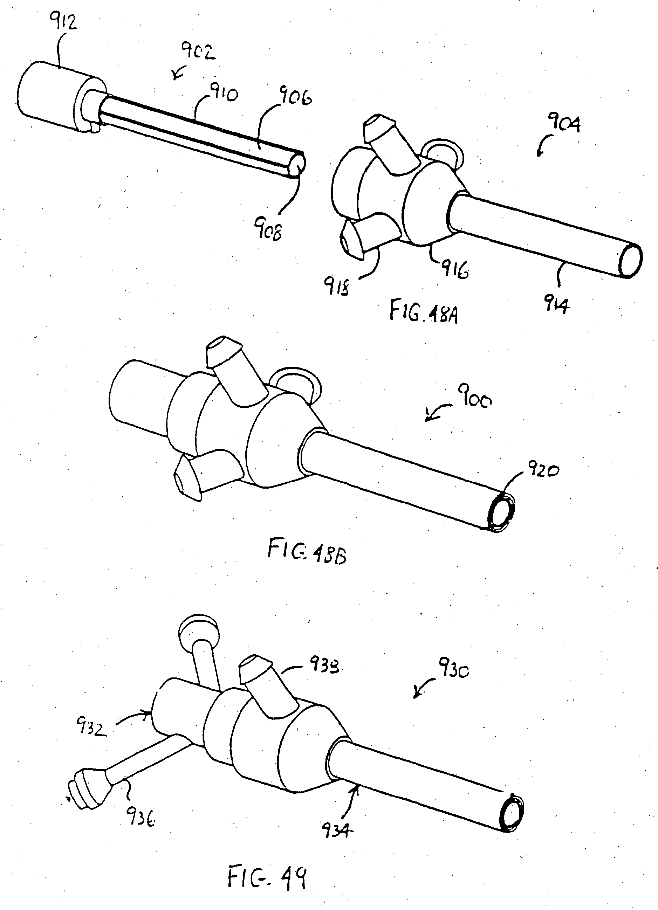

Another alternative embodiment of the present invention forms multi-lumen access device by a combination of a single lumen introducer with a hollow obturator. FIGS. 48A and 48B illustrate a multi-lumen access device 900 comprising an elongated implement, for example a multi-channel obturator 902, inserted into a single lumen sheath or introducer 904. The obturator 902 includes a sheath 906 having a device lumen 908 and, in one preferred embodiment, three evenly circumferentially arranged longitudinal ribs 910 extending radially from a proximal end to a distal end of the sheath 906. Any number of the radially extending ribs is within the scope of the present invention. Similarly, the ribs does not have to be arranged evenly circumferentially. A hemostasis valve 912 (within housing) is connected to the proximal end of the sheath 906. The introducer 904 includes a single lumen sheath 914 connected to a hemostasis valve 916 (within housing) with three access ports 918 for infusion of fluids.

When the obturator 902 is inserted into the introducer 904, as shown in Fig. 48B, the ribs 910 contact the inner wall of the introducer sheath 914 and form three (or any other desired number) auxiliary lumens 920. Each auxiliary lumen 920 communicates with the corresponding access port 918 of the introducer 904. To provide a liquid tight seal at the interface between the ribs 910 and inner wall of the introducer sheath 914, the obturator sheath 906 is made from a sufficiently rigid material and is sufficiently sized while the introducer sheath 914 is made from a sufficiently resilient material. Thus, the access device 900 has multiple independent fluid entries and multiple independent lumens. In addition, the obturator may be used as a fluid delivery lumen by having an obturator without a hemostasis valve. The multi-lumen access device 900 should have at least two auxiliary lumens 920, and preferably three, though other numbers of lumens are also within the scope of the present invention.

One of the advantages of the access device 900 over known introducer products is that it provides greater flexibility of use and eliminates the need for a central venous catheter (CVC). The prior art introducer is inserted into the patient; and if another independent lumen is required, a CVC is usually inserted into the patient. By using the access device 900, the introducer 904 is inserted into the patient and if another independent lumen is required as well as a device lumen, the obturator 902 may be inserted into the introducer 904 to achieve multi-lumen access with only one patient entrance site.

FIG. 49 illustrates another multi-lumen access device 930 which is similar to the access device 900 shown in FIGS. 48A and 48B with the exception that an obturator 932 has two access ports 936 for infusion of fluids, and a single lumen introducer 934 has only one access port 938. This arrangement allows all or some of the fluid to be introduced via the obturator 932 instead of the introducer 934. The remaining elements of the access device 930 are not discussed because they are essentially the same as the elements shown in FIGS. 48A and 48B.

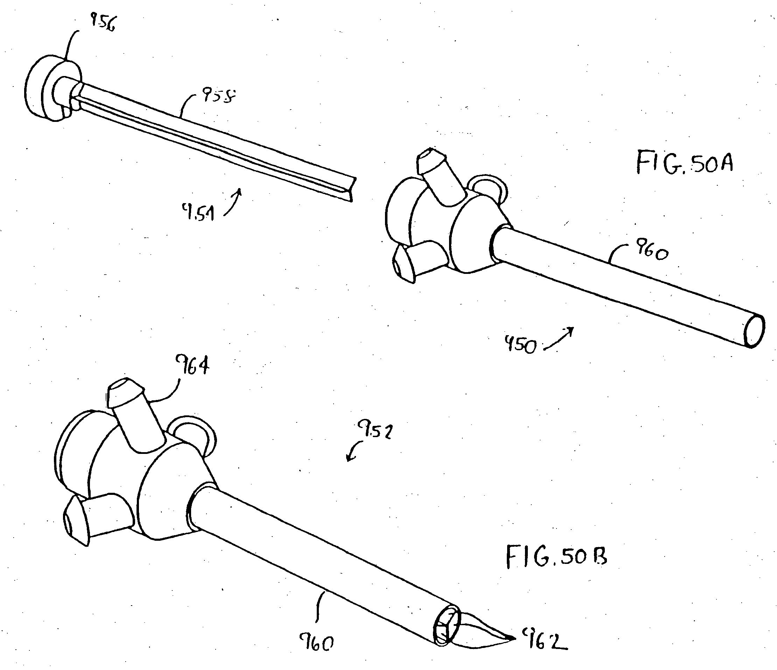

A further alternative MLAD using a solid obturator or solid elongated implement is shown in FIGS. 50A and 50B. In this embodiment, a single lumen catheter or introducer 950 is converted to a multiple lumen access device 952 upon combination with an obturator 954. Obturator 954 comprises a proximal hub 956 and an elongated trefoil portion 958 that closely fits within a sheath 960 of the introducer 950. Three exemplary auxiliary lumens 962 are thus formed within the sheath 960. Three infusion ports 964 provide access to the lumens 962, and any one of them may be adapted to introduce a device through the introducer 950.

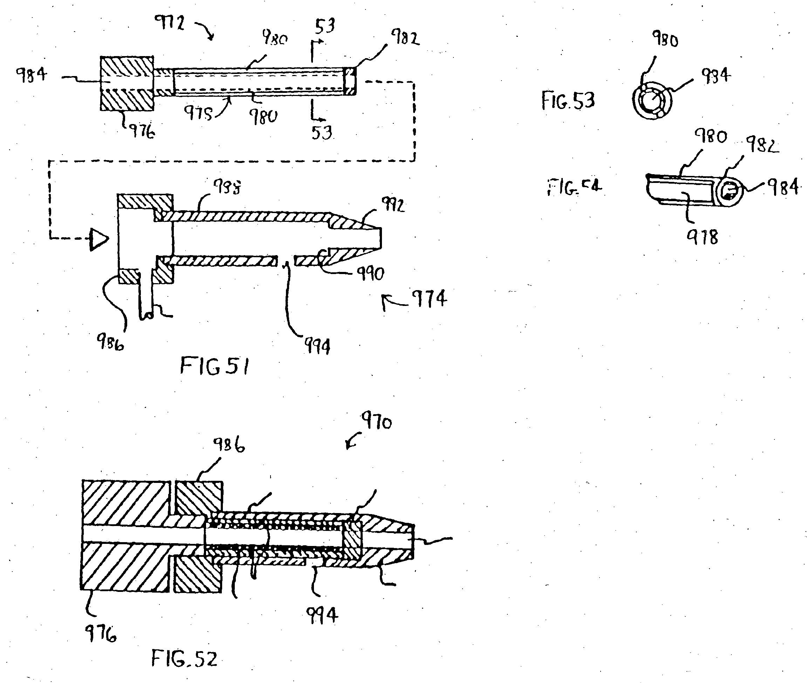

FIGS. 51-54 illustrate a still further MLAD embodiment of the present invention formed using an obturator within an introducer. Specifically, a MLAD 970 is formed by the combination of a hollow obturator 972 with an introducer 974. The obturator includes a proximal hub 976 and a distal tube 978 having a plurality of outwardly directed ribs 980. A distal plug member 982 has a diameter the same as the ribs 980. The obturator 972 defines a hollow through bore extending through the proximal hub 976 and distal tube 978. The introducer 974 includes a proximal hub 986 and distal sheath 988, and also defines a hollow bore therethrough that transitions from a larger proximal diameter to a smaller distal diameter at a step 990. The sheath 988 has a tapered distal tip 992 and an outlet port 994 in one side. A fluid infusion port 996 is provided in the hub 986.

The distal tube 978 fits closely within the sheath 988 , as seen in FIG. 52, until the plug member 982 abuts the internal step 990. The ribs 980 seal against the interior of the bore of the sheath 988 and thus three sealed fluid flow channels are formed between the obturator 972 and introducer 974. Either multiple outlet ports 994 may be provided, one for each channel, of the obturator may be rotated to place one of the three channels into communication with a single outlet port. Devices or other implements can be inserted through the bore 984 while fluid is infused through the channels. Another difference between this embodiment and those previously described is the provision of the tapered distal tip 992 on the introducer 974 that facilitates insertion over a dilator and into a vessel.

Having thus described exemplary embodiments of the present invention, it should be noted by those skilled in the art that the disclosures herein are exemplary only. Accordingly, the present invention is not limited to the specific embodiments as illustrated herein.

## Claims

1. A multiple lumen access device (860), comprising:
a first introducer (862) comprising a tubular single lumen sheath (866) having proximal and distal ends, the first introducer (862) having a valve associated with its lumen; and
a second introducer (864) comprising an elongated implement having a valve associated with its lumen and sized to fit coaxially within the first introducer sheath (866) to thereby form multiple independent valved lumens.

2. The multiple lumen access device (900, 930) of claim 1, further including at least one infusion port (918, 938) being on a proximal end of the multiple lumen access device (900, 930), wherein one of the multiple independent valved lumens is in fluid communication with the infusion port (900, 930).

3. The multiple lumen access device (930) of claim 2, wherein the infusion port (936) is on a proximal end of the second introducer and is in fluid communication with the lumen thereof.

4. The multiple lumen access device of claim 1, wherein each of the introducers includes a hub having a connection site to connect said at least two single lumen introducers.

5. The multiple lumen access device of claim 1. wherein said second introducer (864) sized to fit coaxiary within the first introducer sheath (866) is made from a sufficiently flexible material so that at least one independent lumen formed by said coaxially fitted introducers has flexible walls defined by the flexible material and movable from a relaxed position, wherein at least one independent valved lumen has a first cross-sectional area, to flexed positions wherein the independent valved lumen lumen has cross-sectional areas which are greater than or less than the first cross-sectional area.

6. The multiple lumen access device of claim 5, wherein the second introducer includes a rigid distal tip to facilitate coaxial passage within the first introducer.

7. The multiple lumen access device of claim 1, further including a third introducer comprising a second elongated implement having a valve associated with its lumen and sized to fit coaxially within the second introducer to thereby form another independent valved lumen.

8. The multiple lumen access device (900, 930, 970) of claim 1, further comprising:
at least one infusion port (918, 938) being provided on the proximal end of the first introducer sheath (914, 934); and
wherein the elongated implement of the second introducer (902, 932) comprises a plurality of ribs (910) extending radially outward and the first introducer sheath (914, 934) has an inner wall and wherein said ribs contact the inner wall to form multiple independent lumens (920) therein when said elongated implement is coaxially inserted within said first introducer sheath (914, 934), at least one of the lumens being in fluid communication with the infusion port (918).

9. The multiple lumen access device (900) of claim 8, wherein a liquid tight seal is formed at an interface between the ribs (910) and the inner wall of the first introducer sheath (914).

10. The multiple lumen access device (930) of claim 8, wherein the elongated implement of the second introducer (932) incudes a hub connected to a proximal end, and wherein the hub has two fluid access ports (936).

11. The multiple lumen access device (900) of claim 8, further comprising:
a hub on the proximal end of the first introducer sheath enclosing the valve associated with its lumen.

12. The multiple lumen access device (900) of claim 11, wherein there are multiple infusion ports (918) connected to the first introducer sheath hub (914), and each of the multiple independent valved lumens (920) separately communicate with one of the infusion ports (918).

13. The multiple lum access device (930) of claim 8, wherein said elongated implement further includes a tube (978) about which the ribs (910) project, the tube defining a primary lumen and the ribs (910) contact the inner wall of the first introducer sheath to form a plurality of auxiliary lumens (920).

14. The multiple lumen access device (970) of claim 13, wherein the elongated implement further includes a distal plug member (982) having approximately the same diameter as the ribs, wherein the first introducer sheath lumen transitions from a larger proximal diameter to a smaller distal diameter at a step, and wherein the elongated implement fits closely within the sheath until the plug member (970) abuts the internal step thus forming multiple fluid flow channels between the tube (978) and ribs (980) of the elongated implement and the first introducer sheath lumen.

15. The multiple lumen access device of claim 14, further including an outlet port (994) formed in the first introducer sheath in fluid communication with at least one of the fluid flow channels.

16. The multiple lumen access device of claim 15, wherein the elongated implement may be rotated within the first introducer sheath lumen to place different ones of the fluid flow channels into communication with the outlet port (994).

17. The multiple lumen access device of claim 15, wherein there are multiple outlet ports (994) formed in the first introducer sheath each in communication with a different fluid flow channel.

18. The multiple lumen access device of claim 14, wherein the first introducer sheath (974) has a tapered distal tip (992) that facilitates insertion over a dilator and into a vessel.

## Patentansprüche

1. Zugangseinrichtung (860) mit mehreren Lumen mit:
einer ersten Einführeinrichtung (862) mit einer rohrförmigen Hülle (866) mit einem einzigen Lumen, die ein proximales und ein distales Ende besitzt, wobei die erste Einführeinrichtung (862) ein seinem Lumen zugeordnetes Ventil besitzt, und
einer zweiten Einführeinrichtung (864) mit einem länglichen Werkzeug, welche oder welches ein Ventil besitzt, welches dessen oder deren Lumen zugeordnet ist und geeignet bemessen ist, um koaxial in die Hülle (866) der ersten Einführeinrichtung zu passen, wodurch mehrere unabhängig mit Ventilen verbundene, ausgestattete oder betätigte Lumen gebildet sind.

2. Zugangseinrichtung (900, 930) mit mehreren Lumen nach Anspruch 1, mit zumindest einem Infusions-Anschluss (918, 938), welcher sich an einem proximalen Ende der Zugangseinrichtung (900, 903) mit mehreren Lumen befindet, wobei eines der mehreren unabhängig mit Ventilen verbundene, ausgestattete oder betätigte Lumen fluidisch mit dem Infusions-Anschluss (900, 930) kommuniziert.

3. Zugangseinrichtung (930) mit mehreren Lumen nach Anspruch 2, wobei sich der Infusions-Port (936) an einem proximalen Ende der zweiten Einführeinrichtung befindet und mit deren Lumen fluidisch kommuniziert.

4. Zugangseinrichtung mit mehreren Lumen nach Anspruch 1, wobei jede der Einführeinrichtungen eine Narbe besitzt mit einer Verbindungsstelle zur Verbindung der zumindest zwei Einführeinrichtungen, die lediglich ein einziges Lumen besitzen.

5. Zugangseinrichtung mit mehreren Lumen nach Anspruch 1, wobei die zweite Einführeinrichtung (864), die geeignet bemessen ist, um koaxial in die Hülle (866) der ersten Einführeinrichtung zu passen, aus einem hinreichend flexiblen Material hergestellt ist, so dass zumindest ein unabhängiges Lumen, welches durch die koaxial ineinander passenden Einführeinrichtungen gebildet ist, flexible Wandungen besitzt, die durch das flexible Material definiert oder gebildet sind und bewegbar sind von einer entspannten Position, in welcher zumindest ein unabhängig mit einem Ventil verbundenes, ausgestattetes oder betätigtes Lumen eine erste Querschnittsfläche besitzt, in ausgelenkte Positionen, in denen das unabhängig mit dem Ventil verbundene, ausgestattete oder betätigte Lumen Querschnittsflächen besitzt, die größer sind oder kleiner sind als die erste Querschnittsfläche.

6. Zugangseinrichtung mit mehreren Lumen nach Anspruch 5, wobei die zweite Einführeinrichtung eine steife oder starre distale Spitze besitzt zur Ermöglichung oder Vereinfachung des koaxialen Durchtritts im Inneren der ersten Einführeinrichtung.

7. Zugangseinrichtung mit mehreren Lumen nach Anspruch 1 mit einer dritten Einführeinrichtung, die ein zweites längliches Werkzeug besitzt, welches ein Ventil aufweist, dass dessen oder deren Lumen zugeordnet ist und welche oder welches geeignet bemessen ist, um koaxial in die zweite Einführeinrichtung zu passen, um hierdurch ein weiteres unabhängig mit einem Ventil verbundenes, ausgestattetes oder betätigtes Lumen zu bilden.

8. Zugangseinrichtung (900, 930, 970) mit mehreren Lumen nach Anspruch 1, mit:
zumindest einem Infusions-Anschluss (918, 938), welcher an dem proximalen Ende der Hülle (914, 934) der ersten Einführeinrichtung vorgesehen ist,
wobei das längliche Werkzeug der zweiten Einführeinrichtung (902, 932) mehrere Rippen (910) besitzt, die sich radial auswärts erstrecken, und die Hülle (914, 934) der ersten Einführeinrichtung eine innere Wandung besitzt und wobei die Rippen die innere Wandung kontaktieren zur Bildung mehrerer unabhängiger Lumen (920) darin, wenn das längliche Werkzeug koaxial in die Hülle (914, 934) der ersten Einführeinrichtung eingesetzt ist, wobei zumindest zwei der Lumen fluidisch mit dem Infusions-Anschluss (918) kommunizieren.

9. Zugangseinrichtung (900) mit mehreren Lumen nach Anspruch 8, wobei eine für eine Flüssigkeit dichte Dichtung an einer Zwischenfläche oder Kontaktfläche zwischen den Rippen (910) und der inneren Wandung der Hülle (914) der ersten Einführeinrichtung gebildet ist.

10. Zugangseinrichtung (930) mit mehreren Lumen nach Anspruch 8, wobei das längliche Werkzeug der zweiten Einführeinrichtung (932) eine Narbe aufweist, die mit einem proximalen Ende verbunden ist, und wobei die Narbe zwei fluidische Zugangs-Anschlüsse (936) besitzt.

11. Zugangseinrichtung (900) mit mehreren Lumen nach Anspruch 8 mit:
einer Narbe an dem proximalen Ende der Hülle der ersten Einführeinrichtung, welche das dem Lumen zugeordnete Ventil umschließt oder einschließt.

12. Zugangseinrichtung (900) mit mehreren Lumen nach Anspruch 11, wobei es mehrere Infusions-Anschlüsse (918) gibt, die mit der Narbe der Hülle (914) der ersten Einführeinrichtung verbunden sind, und wobei jedes der mehreren unabhängig mit einem Ventil verbundenen, ausgestatteten oder betätigten Lumen (920) separat mit einem der Infusions-Anschlüsse (918) kommuniziert.

13. Zugangseinrichtung (930) mit mehreren Lumen nach Anspruch 8, wobei das längliche Werkzeug ein Rohr (978) aufweist, um welches Rippen hervorstehen, wobei das Rohr ein primäres Lumen definiert oder bildet und die Rippen (910) die innere Wandung der Hülle der ersten Einführeinrichtung kontaktieren zur Bildung mehrerer Hilfslumen (920).

14. Zugangseinrichtung (970) mit mehreren Lumen nach Anspruch 13, wobei das längliche Werkzeug ein distales Stößel-Element (982) aufweist, welches ungefähr denselben Durchmesser besitzt wie die Rippen, wobei das Lumen der Hülle der ersten Einführeinrichtung bei einer Stufe übergeht von einem größeren proximalen Durchmesser zu einem kleineren distalen Durchmesser und wobei das längliche Werkzeug dicht oder eng in die Hülle passt bis das Stößel-Element (970) an die innere Stufe anstößt, womit mehrere fluidische Fluss-Kanäle zwischen dem Rohr (978) und Rippen (980) des länglichen Werkzeugs und dem Lumen der Hülle der ersten Einführeinrichtung gebildet sind.

15. Zugangseinrichtung mit mehreren Lumen nach Anspruch 14 mit einem Auslass-Anschluss (994), welcher in der Hülle der ersten Einführeinrichtung gebildet ist, der fluidisch mit zumindest einem der fluidischen Fluss-Kanäle kommuniziert.

16. Zugangseinrichtung mit mehreren Lumen nach Anspruch 15, wobei das längliche Werkzeug in dem Lumen der Hülle der ersten Einführeinrichtung verdreht werden kann, um unterschiedliche fluidische Fluss-Kanäle mit dem Auslass-Anschluss (994) kommunizieren zu lassen.

17. Zugangseinrichtung mit mehreren Lumen nach Anspruch 15, wobei es mehrere AuslassAnschlüsse (994) gibt, die in der Hülle der ersten Einführeinrichtung gebildet sind, die jeweils mit einem anderen fluidischen Fluss-Kanal kommunizieren.

18. Zugangseinrichtung mit mehreren Lumen nach Anspruch 14, wobei die Hülle (974) der ersten Einführeinrichtung eine abgeschrägte distale Spitze (992) besitzt, die das Einsetzen über einen Dilator und in ein Gefäß ermöglicht oder vereinfacht.

## Revendications

1. Dispositif d'accès à plusieurs lumières (860), comprenant :
un premier introducteur (862) comprenant une gaine de lumière tubulaire unique (866) ayant des extrémités proximale et distale, le premier introducteur (862) ayant une valve associée à sa lumière ; et
un deuxième introducteur (864) comprenant un accessoire allongé ayant une valve associée à sa lumière et dimensionné pour se placer coaxialement dans la gaine du premier introducteur (866) pour former ainsi plusieurs lumières indépendantes à valve.

2. Dispositif d'accès à plusieurs lumières (900, 930) selon la revendication 1, comprenant en outre au moins un port de perfusion (918, 938) qui se situe sur une extrémité proximale du dispositif d'accès à plusieurs lumières (900, 930), dans lequel l'une des plusieurs lumières indépendantes à valve est en communication de fluide avec le port de perfusion (900, 930).

3. Dispositif d'accès à plusieurs lumières (930) selon la revendication 2, dans lequel le port de perfusion (936) est situé sur une extrémité proximale du deuxième introducteur et est en communication de fluide avec sa lumière.

4. Dispositif d'accès à plusieurs lumières selon la revendication 1, dans lequel chacun des introducteurs comprend un raccord ayant un site de raccordement pour relier lesdits au moins deux introducteurs de lumière unique.

5. Dispositif d'accès à plusieurs lumières selon la revendication 1, dans lequel ledit deuxième introducteur (864) dimensionné pour s'adapter coaxialement dans la gaine de premier introducteur (866) est constitué d'un matériau suffisamment flexible de sorte qu'au moins une lumière indépendante formée par lesdits introducteurs coaxialement adaptés possède des parois flexibles définies par le matériau flexible et mobile depuis une position détendue, où au moins une lumière indépendante à valve a une première aire en coupe, à des positions fléchies où la lumière indépendante à valve a des aires en coupe qui sont supérieures ou inférieures à la première aire en coupe.

6. Dispositif d'accès à plusieurs lumières selon la revendication 5, dans lequel le deuxième introducteur comprend une pointe distale rigide pour faciliter le passage coaxial dans le premier introducteur.

7. Dispositif d'accès à plusieurs lumières selon la revendication 1, comprenant en outre un troisième introducteur comprenant un second accessoire allongé ayant une valve associée à sa lumière et dimensionné pour s'adapter coaxialement dans le deuxième introducteur pour former ainsi une autre lumière indépendante à valve.

8. Dispositif d'accès à plusieurs lumières (900, 930, 970) selon la revendication 1, comprenant en outre :
au moins un port de perfusion (918, 938) qui est disposé sur l'extrémité distale de la gaine de premier introducteur (914, 934) ; et
dans lequel l'accessoire allongé du deuxième introducteur (902, 932) comprend une pluralité de nervures (910) s'étendant radialement vers l'extérieur et la gaine de premier introducteur (914, 934) a une paroi interne et dans lequel lesdites nervures entrent en contact avec la paroi interne pour former plusieurs lumières indépendantes (920) à l'intérieur lorsque ledit accessoire allongé est inséré coaxialement dans ladite gaine de premier introducteur (914, 934), au moins l'une des lumières étant en communication de fluide avec le port de perfusion (918).

9. Dispositif d'accès à plusieurs lumières (900) selon la revendication 8, dans lequel un joint étanche au liquide est formé au niveau d'une interface entre les nervures (910) et la paroi interne de la gaine de premier introducteur (914).

10. Dispositif d'accès à plusieurs lumières (930) selon la revendication 8, dans lequel l'accessoire allongé du deuxième introducteur (932) comprend un raccord relié à une extrémité proximale, et dans lequel le raccord a deux ports d'accès de fluide (936).

11. Dispositif d'accès à plusieurs lumières (900) selon la revendication 8, comprenant en outre :
un raccord sur l'extrémité proximale de la gaine de premier introducteur enfermant la valve associée à sa lumière.

12. Dispositif d'accès à plusieurs lumières (900) selon la revendication 11, dans lequel se trouvent des ports de perfusion multiples (918) reliés au raccord de gaine de premier introducteur (914), et chacune des plusieurs lumières indépendantes à valve (920) communique séparément avec l'un des ports de perfusion (918).

13. Dispositif d'accès à plusieurs lumières (930) selon la revendication 8, dans lequel ledit accessoire allongé comprend en outre un tube (978) autour duquel les nervures (910) font saillie, le tube définissant une lumière primaire et les nervures (910) entrent en contact avec la paroi interne de la gaine de premier introducteur pour former une pluralité de lumières auxiliaires (920).

14. Dispositif d'accès à plusieurs lumières (970) selon la revendication 13, dans lequel l'accessoire allongé comprend en outre un organe de bouchon distal (982) ayant approximativement le même diamètre que les nervures, dans lequel la lumière de gaine de premier introducteur passe d'un diamètre proximal plus grand à un diamètre distal plus petit à un étage, et dans lequel l'accessoire allongé s'adapte fermement dans la gaine jusqu'à ce que l'organe de bouchon (970) vienne en butée contre l'étage interne formant ainsi plusieurs canaux d'écoulement de fluide entre le tube (978) et les nervures (980) de l'accessoire allongé et la lumière de gaine de premier introducteur.

15. Dispositif d'accès à plusieurs lumières selon la revendication 14, comprenant en outre un port de refoulement (994) formé dans la gaine de premier introducteur en communication de fluide avec au moins l'un des canaux d'écoulement de fluide.

16. Dispositif d'accès à plusieurs lumières selon la revendication 15, dans lequel l'accessoire allongé peut être tourné dans la lumière de gaine de premier introducteur pour placer différents canaux des canaux d'écoulement de fluide en communication avec le port de refoulement (994).

17. Dispositif d'accès à plusieurs lumières selon la revendication 15, dans lequel se trouvent plusieurs ports de refoulement (994) formés dans la gaine de premier introducteur chacun en communication avec un canal d'écoulement de fluide différent.

18. Dispositif d'accès à plusieurs lumières selon la revendication 14, dans lequel la gaine de premier introducteur (974) a une pointe distale effilée (992) qui facilite l'insertion sur un dilatateur et dans un vaisseau.
